# EUROPEAN PATENT APPLICATION

(11) **EP 4 623 806 A1**
(43) Date of publication of application: **01.10.2025**
(21) Application number: 25165344.0
(22) Date of filing: 21.03.2025
(51) Int. Cl.: A61B 5/00, A61B 5/318, G16H 40/67

(54) **NETWORK REPRESENTATION OF DIAGNOSTIC ECG SIGNALS**

(30) Priority: 28.03.2024 US 202463571255 P
(71) Applicant: Draeger Medical Systems, Inc., Andover, MA 01810 (US)
(72) Inventor: RISHER-KELLY, Clifford M, Wells, 04090 (US); ZHANG, Ruwen, Andover, 01810 (US); FALLON, Joseph M, Brunswick, 04011 (US); FORDE, Ryan, Salem, 03079 (US)
(74) Representative: Haseltine Lake Kempner LLP

(57) **Abstract**

A technique described herein reduces congestion and traffic on the computing system of a clinical care system, thereby easing problems created by such congestion and traffic. Diagnostic Quality ECG signals are transmitted over a computer system by a provider device. Monitor Quality signals consumed by consumer devices of the clinical care system may be derived from the Diagnostic Quality signals on the consumer side. Thus, only the Diagnostic Quality signals need be transmitted over the computing system. (In some embodiments the Monitor Quality signals may nevertheless be transmitted over the computing network should this be desired.) The bandwidth of the computing system therefore need only accommodate the Diagnostic Quality signals. Autoblocking on both the provider and consumer sides of the clinical care system prevents, or at least inhibits, the introduction of undesirable artifacts in the transmitted Diagnostic Quality signals that would otherwise be present due to the transmission.

## Description

### TECHNICAL FIELD

The present disclosure relates generally to the field of medical monitoring of physiological parameters in a patient and, more particularly, to monitoring a patient's condition using an electrocardiogram ("ECG").

### BACKGROUND

This section of this document introduces information about and/or from the art that may provide context for or be related to the subject matter described herein and/or claimed below. It provides background information to facilitate a better understanding of the various aspects of the present invention. This is a discussion of "related" art. That such art is related in no way implies that it is also "prior" art. The related art may or may not be prior art. The discussion in this section of this document is to be read in this light, and not as admissions of prior art.

Conventional, modern medical practice frequently includes electronic measurement and/or monitoring of a patient's physiological condition. Commonly known physiological parameters that are measured and/or monitored might include, for example and without limitation, blood pressure, body temperature, vision acuity, quantities of various substances in the blood, etc. Because of the value imparted by this information, the art of medical monitoring has developed an array of instruments and procedures for acquiring this kind of information.

One well-known measurement/monitoring technique is an "electrocardiogram" ("ECG"). Electrocardiograms are commonly used to monitor patients' heart conditions as well as to analyze, detect, and/or predict cardiac events and conditions. In clinical settings, ECG signals representative of a patient's condition are captured in waveforms and analyzed by physiological monitoring devices called ECG monitors. However, ECGs are also useful for acquiring other kinds of medical information besides cardiac events and conditions.

An ECG graphs voltage acquired from a person's body over time. The voltages represent electrical activity of the heart that cause the heart to beat. To acquire the voltages, sensors, or "electrodes", are placed at selected points on the person's body. The number and location of the electrodes are fairly standardized but may vary depending on the type of medical information that is of interest. For example, in one common nomenclature, the sensors are electrically connected to the physiological monitoring device by cables. The sensors and cables together are then colloquially referred to as "leads" in a clinical setting. For present purposes, the ECG sensors and cables together shall be referred to as "ECG lead assemblies" rather than "leads".

There are a variety of ECG configurations defined by the arrangement and placement of the sensors on the patient's body. In the context of these configurations, those in the art frequently referred to ECG "leads". An ECG lead is derived by analyzing multiple electrodes or sensors, e.g. limb lead is computed per two electrode signals while chest / augmented leads based on one electrode and Wilson Center (3 electrode potential average). Note that the discussion of these configuration and their ECG "leads" does not reference the number of cables. That is because the number of cables does not define the "leads" of the ECG configuration. For example, one particular type of ECG configuration is what is known as a "12-lead ECG", in which a 12 lead configuration uses 10 electrodes or 10 potentials are measured. In the label "12-lead ECG", the "12-lead" portion refers to the derived pairs generated from the 10 electrodes.

In clinical settings, ECG signals representative of a patient's condition are captured in waveforms and analyzed by physiological monitoring devices. In clinical settings, however, noise contamination caused by artifact signals adversely impacts the precision and accuracy in ECG signal analysis. A variety of sources may cause artifact signals, including physiological artifacts caused by patients and non-physiological artifacts caused by electric circuitry in the physiological monitoring devices and/or other devices in the clinical environment. Thus, it is important for physiological monitoring devices to accurately detect artifact signals, identify and analyze the corrupted segments of ECG signals contaminated by artifacts.

One aspect of ECG analysis is the resolution, or quality, of the ECG signal being analyzed. Signal quality in this context may include both time resolution (sampling rate) and amplitude resolution (in digitization). Commonly, as the analog waveforms generated by the sensors are received over the cables of the lead assemblies by an ECG monitor, they are converted to digital form. This includes a sampling of the acquired, or "raw", ECG signals. For some uses, the acquired ECG signals are sampled at 250 samples per second ("sps"), and this is called "Monitor Quality" in the art. For some other uses, the acquired ECG signals are sampled at 500 sps, and this is called "Diagnostic Quality" in the art.

As the name implies, Monitor Quality ECG signals may be used to monitor the cardiac activity of the patient, particularly in real-time or near real-time. Such monitoring typically occurs at the patient's bedside or at a central monitoring station, such as a nurse's station on the floor of a medical facility. The higher resolution Diagnostic Quality ECG signals are used for certain diagnostic analyses to detect adverse cardiac events in a patient. These diagnostic analyses may be performed after the cardiac event has occurred and at a location remote from the patient's bedside.

The physiological monitoring device, such as an ECG monitor, by which the ECG signals are acquired and the device on which the diagnostics are run, such as a medical-grade workstation like a central station or a review station, typically are using part of a larger computing and communications system. Diagnostic quality ECG signals are used in real time to perform 12-lead rest ECG reports or in analyzing S-T segments. Currently, Diagnostic Quality data is typically transmitted over the computing system to the medical-grade workstation in "snapshots". The stored data, however, is insufficient in quality and quantity to run analyses such as 12-lead reports or S-T segment analysis at later dates.

### SUMMARY

A technique is disclosed that facilitates certain historical analyses, such as a 12 lead analysis on historical data, while reducing congestion and traffic on the computing system of a clinical care system. The technique thereby eases problems created by such congestion and traffic. In order to be able to do perform such historical analyses, higher fidelity data is needed on the system. The easy way to solve the problem is to send both Monitor Quality (e.g., 250 sps) data and Diagnostic Quality (e.g., 500 sps) data on the wire continuously. This approach increases traffic and congestion on the computing system. The presently disclosed technique reduces this traffic and congestion by combining both streams of data.

More particularly, Diagnostic Quality ECG signals are transmitted over a computer system by a provider device. Monitor Quality signals consumed by the consumer devices are derived from the Diagnostic Quality signals on the consumer side. Thus, only the Diagnostic Quality signals need be transmitted over the computing system. (Note, however, that in some embodiments the Monitor Quality signals may nevertheless be transmitted over the computing network should this be desired.) The bandwidth of the computing system therefore need only accommodate the Diagnostic Quality signals. Autoblocking, sometimes also called baseline restoration, filters on both the provider and consumer sides of the clinical care system prevents, or at least inhibits, the introduction of undesirable artifacts in the transmitted Diagnostic Quality signals that would otherwise be present due to the transmission.

Thus, in a first aspect, a computer-implemented method comprises: autoblocking a plurality of acquired electrocardiogram ("ECG") signals sampled at a Diagnostic Quality rate by an ECG signal provider device to generate a first set of autoblocked Diagnostic Quality ECG signals; transmitting the first set of autoblocked Diagnostic Quality ECG signals from the ECG signal provider device over a computing system; autoblocking the transmitted first set of autoblocked Diagnostic Quality ECG signals at a first ECG signal consumer device to generate a second set of Diagnostic Quality ECG signals; and consuming the second set of twice autoblocked Diagnostic Quality ECG signals.

In a second aspect, a clinical care system comprises a computing system, an ECG signal provider device, and an ECG signal consumer device. The ECG signal provider device is programmed to: autoblock a plurality of acquired electrocardiogram ("ECG") signals sampled at a Diagnostic Quality rate to generate a first set of autoblocked Diagnostic Quality ECG signals; and transmit the first set of autoblocked Diagnostic Quality ECG signals from the ECG signal provider device over the computing system. The ECG signal consumer device is programmed to: obtain the transmitted first set of autoblocked Diagnostic Quality ECG signals received over the computing system; autoblock the transmitted plurality of Diagnostic Quality ECG signals at a first ECG signal consumer device to generate a plurality of Monitor Quality ECG signals; and consume at least one of the plurality of the Diagnostic Quality ECG signals and the Monitor Quality ECG signals.

In a third aspect, a method for use in monitoring the physical condition of a patient is substantially as shown and described.

In a fourth aspect, a physiological monitoring device is substantially as shown and described.

In a fifth aspect, an electrocardiogram ("ECG") consumer device is substantially as shown and described.

In a sixth aspect, a clinical care system substantially as shown and described.

The above presents a simplified summary of the invention as claimed below in order to provide a basic understanding of some aspects of the invention. This summary is not an exhaustive overview of the invention. It is not intended to identify key or critical elements of the invention or to delineate the scope of the invention. Its sole purpose is to present some concepts in a simplified form as a prelude to the more detailed description that is discussed later.

### BRIEF DESCRIPTION OF THE DRAWINGS

In the drawings, like reference numbers generally indicate identical, functionally similar, and/or structurally similar elements.
FIG. 1 is a schematic diagram of an example of a clinical care system including an ECG signal provider, a computing system, and an ECG signal consumer according to an embodiment of the present disclosure.
FIG. 2 illustrates a method by which, in accordance with one or more embodiments, Diagnostic Quality ECG signals are transmitted over a computing system for use by a consuming device.
FIG. 3A-FIG. 3B conceptually depict two particular implementations of the clinical care system of FIG. 4A.
FIG. 4A-FIG. 4B schematically illustrate ECG signal provider device implementations according to one or more examples.
FIG. 5A-FIG. 5B schematically illustrate ECG signal provider consumer implementations according to one or more examples.
FIG. 6 illustrates a computer-implemented method for use in monitoring the physical condition of a patient in a clinical care system.
FIG. 7 schematically depicts one particular implementation of a provider device and one particular implementation of a consumer device in the context of a particular clinical care system.
FIG. 8A-FIG. 8B illustrate autoblocking techniques employed in the computer-implemented method of FIG. 6 in the particular clinical care system of FIG. 7.
FIG. 9A-FIG. 9B illustrate the contents and makeup of a generalized packet of data packetized from a Diagnostic Quality ECG signal for transmission over a computing system in accordance with one or more embodiments.
FIG. 10 illustrates a particular embodiment of a clinical care system in which technique described herein may be implemented in one or more embodiments.
FIG. 11A-FIG 11B illustrate the efficacy of the technique disclosed herein.

While the disclosed subject matter is susceptible to various modifications and alternative forms, the drawings illustrate specific implementations described in detail by way of example. It should be understood, however, that the description herein of specific examples is not intended to limit that which is claimed to the particular forms disclosed, but on the contrary, the intention is to cover all modifications, equivalents, and alternatives falling within the scope of the appended claims.

### DETAILED DESCRIPTION

Illustrative examples of the subject matter claimed below are disclosed. In the interest of clarity, not all features of an actual implementation are described for every example in this specification. It will be appreciated that in the development of any such actual implementation, numerous implementation-specific decisions may be made to achieve the developers' specific goals, such as compliance with system-related and business-related constraints, which will vary from one implementation to another. Moreover, it will be appreciated that such a development effort, even if complex and time-consuming, would be a routine undertaking for those of ordinary skill in the art having the benefit of this disclosure.

As alluded to above, the distinction between Diagnostic Quality ECG signals and Monitor quality ECG signals can present a number of issues in a clinical care environment. Monitor Quality ECG signals may be readily transmitted from the patient's bedside to remote locations. However, there are some kinds of analyses that require Diagnostic Quality ECG signals. The transmission of Diagnostic Quality ECG signals during baseline restoration introduces undesirable artifacts into the signals. Furthermore, transmitting both Diagnostic Quality ECG signals and Monitor Quality ECG signals congests the computing networks, thereby limiting the number of devices being able to work in the ecosystem and slowing transmission times and requiring increased computational capacity.

The technique disclosed herein transmits Diagnostic Quality ECG signals over a computing system without introducing undesirable artifacts. More particularly, one significant aspect of ECG signal processing is a technique called "autoblocking", which is used to correct for a phenomenon known as "baseline wander". In conventional practice, autoblocking is performed at the point of acquisition of the ECG signals rather than at the point of consumption. Attempts to move the autoblocking from the point of acquisition to the point of consumption in order to transmit the ECG signals at Diagnostic Quality introduced the undesirable artifacts.

More particularly, autoblocking is a technique performed on Monitor Quality ECG signals to temporarily change a high pass filter frequency from 0.67 Hz to 5 Hz. This has the effect of forcing the baseline of the signal to zero, thereby ensuring that the signal is on the display screen and is being displayed. This is generally performed at the point of acquisition prior to the transmission over the computing system. Performing autoblock on a Diagnostic Quality signal (0.05 Hz to 150 Hz) instead of the Monitor Quality signal (0.67 Hz to 40 Hz) generated 10 times as many autoblocking events. This was expected due to the 10 times change in lower frequency content, so the approach was fine-tuned to minimize the amount of autoblocking. However, this fine tuning generated the undesirable artifacts, which are undesirable because they are not clinically acceptable. The artifact is due to filtering at the point of consumption at 0.067 Hz when the signal was being autoblocked at 5 Hz at the point of acquisition.

The presently disclosed technique permits retrospective ECG analysis on stored, Diagnostic Quality waveforms while maintaining the ability to display Monitor Quality waveforms from the same data. The technique is disclosed in the context of one or more embodiments in which Diagnostic Quality ECG signals are transmitted over a computing system from the point of acquisition to another point remote therefrom. At the remote point, the Diagnostic Quality signals may be analyzed, stored, or archived. Note that, for embodiments in which the Diagnostic Signals are stored, any filtering and/or processing as described herein is performed prior to storage rather than upon retrieval. Furthermore, such storage is one form of consumption within the present context and the storage device can consequently be considered a consumer device.

The Diagnostic Quality ECG signals can be accessed and analyzed for as long as they are stored and archived. The analyses can include, but are not limited to, 12-lead rest ECG reports or S-T segment analysis, for example. The Diagnostic Quality ECG signals can also be accessed and filtered to generate Monitor Quality ECG signals. The Monitor Quality signals can be used as desired, including, but not limited to, display, for example.

The technique will be disclosed herein in the context of particular embodiments. However, the technique is not limited to these particular embodiments. It is to be understood that the technique can be applied to monitoring and/or treatment context in which ECG signals are acquired, displayed, transmitted, and/or analyzed. Those in the art having the benefit of this disclosure will be able to readily adapt the disclosed technique to contexts other than those disclosed herein.

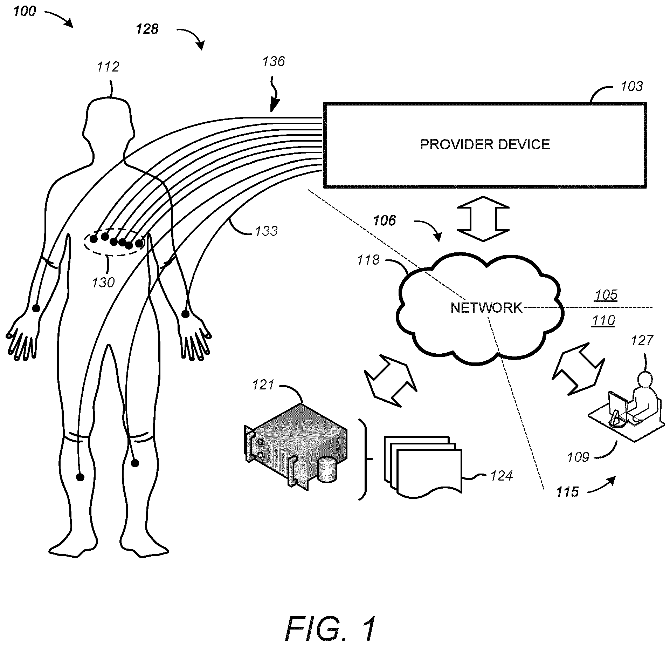

Turning now to the drawings, FIG. 1 is a schematic diagram of an example of a clinical care system 100. The clinical care system 100 includes an ECG signal provider device 103, a computing system 106, and an ECG signal consumer device 109 according to an embodiment of the present disclosure. The provider device 103 and the consumer device 109 define what may be considered as the provider side 105 and the consumer side 110 of the clinical care system 100. The provider device 103 is so-called because it provides acquired ECG signals across various connections to other devices. Similarly, the consumer device 109 is so-called because it consumes acquired ECG signals provided by other devices over various connections. Those connections will be discussed further below.

In the illustrated embodiment, the provider device 103 is a physiological monitoring device and, more particularly, an ECG monitor. The provider device 103 is located locally to the patient 112, typically at a bedside or, at least, in the same room so that the ECG signals may be acquired. The consumer device 109 is located remotely from the patient 112, for example, outside the patient's room at a remote location 115, such as a central monitoring station (not otherwise shown) or a nursing station. The consumer device 109 may be, for instance, a desktop computer or a workstation in this particular implementation.

However, the claimed subject matter is not limited to the illustrated embodiment. The provider device 103 may be any medical device that provides acquired ECG signals to other devices. Typically, the provider device 103 will also be the device by which the ECG signals are acquired although this is not required. The consumer device 109 may be any kind of computing device known to the art that has sufficient computing power to perform the tasks assigned to it within the scope of this disclosure. In various embodiments, the consumer device 109 may be a desktop computing device, a workstation, a terminal computing device, a laptop computing device, a personal computer, a tablet, a mobile communications device (e.g., a mobile phone), *etc.*

While the provider device 103 is located locally relative to the patient, the consumer device 109 may be located virtually anywhere remote from the patient 106 so long as the requisite connectivity is present. For example, instead of a central monitoring station, the consumer device 109 may be located in an attending physician's office in another building in some embodiments. As used herein, whether a location is "local" or "remote" depends on proximity to the patient and whether a caregiver for the patient can access the device from the patient's bedside.

The computing system 106 may be a distributed computing environment including a network 118 over which the ECG procedure 128 transmits data and information. The network 118 may include a private network, a public network, or some combination thereof. Thus, in the illustrated embodiment, communications between the provider device 103 and the consumer device 109 implement networking communications protocols. Suitable protocols include, but are not limited to, Transmission Control Protocol/Internet Protocol ("TCP/IP"), User Datagram Protocol/Internet Protocol ("UDP/IP"), and File Transfer Protocol ("FTP"). In particular, the communications in the illustrated embodiments employ TCP/IP.

However, there is no requirement that the computing system 106 include a network such as the network 118. The computing system 106 may, for example, in some embodiments, instead communicate peer-to-peer ("P2P"). These alternative, non-networked embodiments may nevertheless still use some networking communications protocols, such as TCP/IP or other packet switching protocols. However, there also is no requirement that network communication or packet switched communications protocols be used. Some embodiments, for example, may communicate in bit streams in various kinds of available predefined formats.

The communications among the provider device 103, the computing system 106, the consumer device 109, and other resources of the clinical care system 100 may be wireless, wired, or some combination thereof, depending upon the technical capabilities of the various components. Suitable wireless protocols may include, but are not limited to, BLUETOOTH^{®}, cellular telephony, WIFI^{®}, IEEE802.11, Radio Frequency For Consumer Electronics ("RF4CE"), and/or IEEE802.15.4 (e.g., ZIGBEE^{®}). Wired communications may employ the ETHERNET^{®} protocol, but others may also be used.

The clinical care system 100 furthermore includes a storage 121 serving as an electronic records repository populated by a plurality of electronic medical records ("EMRs") 124. The EMRs 124 might include, depending on the implementation and without limitation, medication regimens, diagnosed medical conditions, medical history, historical medical data, etc. Similarly, data sensed by or entered at the provider device 103 may be communicated to, for instance, a respective ERM 124 for the patient 112. This data may include acquired and transmitted ECG signals such as will be discussed more fully below. The data in the EMRs 124 may be stored, or even archived, and later retrieved.

The storage 121 may be, and typically will be, a part of the computing system 106. For example, the computing system 106 may include a cloud, or various cloud resources, not separately shown. The storage 121 may then, in these embodiments, be cloud resources leased and allocated to the computing system 106. Or, the storage 121 may be some kind of mass storage (e.g., a Redundant Array of Independent Disks ("RAID")) located at the facility which the patient 112 and the central monitoring station are located. Those in the art having the benefit of this disclosure will appreciate still other variations regarding the *situs* of the storage 121 relative to the other components of the clinical care system 100.

The respective EMR 124 for the patient 112 includes at least a set of Diagnostic Quality ECG signals. The Diagnostic Quality ECG signals are acquired by the provider device 103, transmitted over the computing system 106 at Diagnostic Quality, and stored in the respective EMR 124. This permits the caregiver 127 at the consumer device 109 to access the EMR 124 and use the Diagnostic Quality ECG signals for, for example, 12-lead rest ECG reports or analyze S-T segments. In some scenarios, the consuming device 109 may receive the transmitted Diagnostic Quality ECG signals directly from the provider device 103 over the computing system 106 without being first being stored.

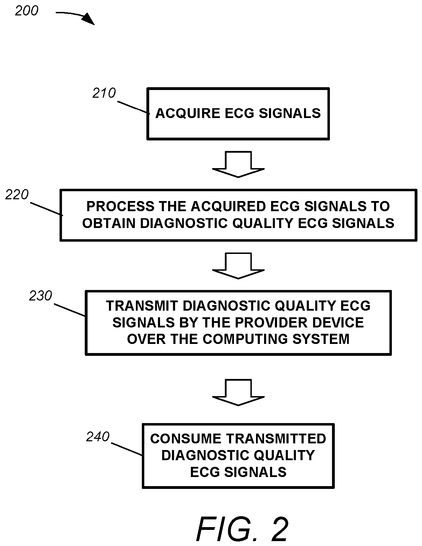

FIG. 2 illustrates a method 200 by which, in accordance with one or more embodiments, is for use in monitoring and/or evaluating the physical condition of a patient using the clinical care system 100 of FIG. 1. In the disclosed method, Diagnostic Quality ECG signals are transmitted over a computing system for use by a consuming device. For present purposes, the method 200 will be discussed in the context of the clinical care system 100 of FIG. 1. However, the method 200 is not limited to the clinical care system 100 and may be practiced with other clinical care systems.

Referring now to FIG. 1 and FIG. 2 collectively, the method 200 begins with acquisition of the raw ECG signals (at 210). This implicitly includes performing an ECG procedure 128. To that end, a plurality of ECG sensors, or electrodes, 130 positioned on the body of the patient 112. Those in the art having the benefit of this disclosure will appreciate that there are many configurations for the ECG sensors 130 by which ECG data may be acquired and, so, the ECG signal acquisition (at 210) is not limited to the illustrated embodiment. The ECG signals may be acquired using any suitable configuration known to the art. The ECG signals generated by the ECG sensors 130 are then transmitted over the cables 133 (only one indicated) of the lead assemblies 136 to the provider device 103.

The method 200 continues by processing (at 220) the acquired ECG signals to obtain Diagnostic Quality ECG signals. One particular technique for processing the acquired ECG signals will be discussed below. However, one defining characteristic of Diagnostic Quality ECG signals is that they are sampled at a rate of 500 sps. This processing (at 220) is performed by the providing device 103. More particular, in the illustrated embodiment, the Diagnostic Quality sample rate is a minimum of 300 samples per second ("sps"). This is the minimum Nyquist sample rate for an acquired ECG signal of 150 Hz. However, to provide an adequate margin over the minimum Nyquist sample rate of 300 sps, the illustrated embodiments employ a Diagnostic Quality rate of 500 sps. This will produce autoblocked, Diagnostic Quality ECG signals between .05-150 Hz.

The Diagnostic Quality ECG signals are then transmitted (at 230) by the provider device 103 over the computing system 106. As discussed above, this transmission can occur using many different kind of technologies in different implementations and embodiments. The transmissions may be wired or wireless, networked or not, and use network communications protocols or not, *etc.* The type and nature of the transmission is limited only by the available technology in any given embodiment.

The transmitted, Diagnostic Quality ECG signals are consumed (at 240). As used herein, the term "consumed", including its variants such as "consume", "consumption", *etc.,* means that the Diagnostic Quality ECG signals are used for some practical application in the furtherance of some practical, pre-existing technology. Practical applications include, but are not limited to, activities such as providing clinical care, academic study, clinical care review, clinical care evaluation, historical review, *etc.* Typically, though not necessarily, this may include some kind of rendering for display at Diagnostic Quality or analyses such as 12-lead reports or S-T segment analysis. The pre-existing technology is the electronic monitoring and/or treatment of patients through their cardiac activities as represented by ECG signals.

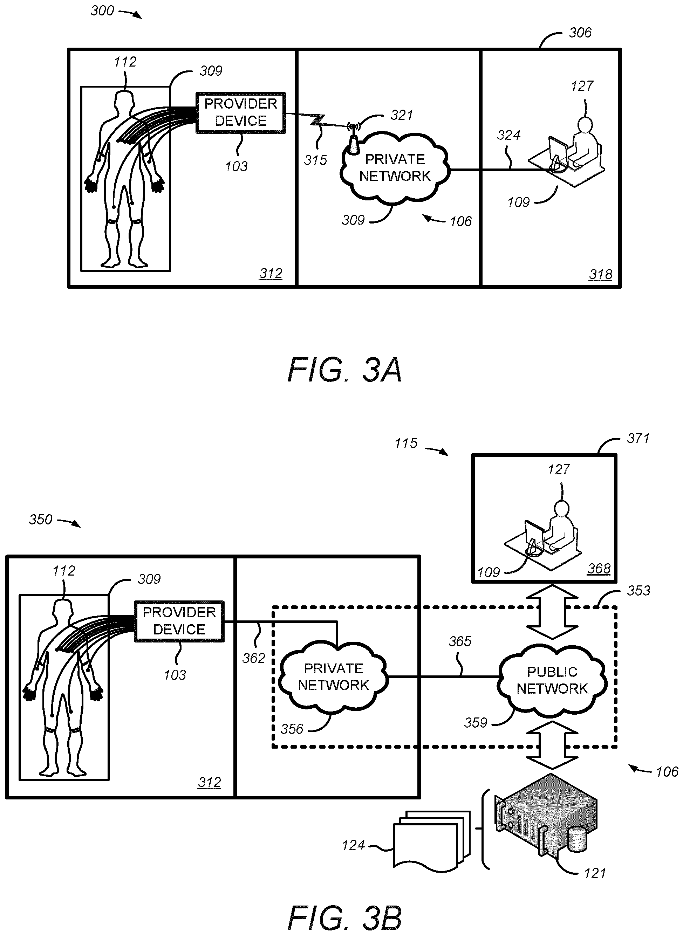

For a first example, in a clinical care system 300 illustrated in FIG. 3A, the network 303 of the computing system 106 may be a private, wired network 303 in a facility 306 in which the patient 112 is receiving care. The provider device 103 is located at the bed 309 in the room 312 of the patient 112 and communicates over the wireless link 315. The consumer device 109 may be located at a nurse's station 318 located remotely from the room 312 on the same floor of the facility 306 with a wired connection 324 to the private network 303. The provider device 103 may wirelessly transmit the Diagnostic Quality ECG signals to a wireless access point 321 that is a part of the private network 303. The Diagnostic Quality ECG signals may be consumed by the consumer device 109 in real-time or near real-time. For example, the caregiver 127 may wish to monitor the ECG activity of the patient 112 in the course of providing clinical care.

Note that what constitutes "real-time" and "near real-time" will be dependent on what is acceptable in clinical care practice. Those in the art have the benefit of this disclosure will appreciate that, regardless of what technologies are used, there will be some time lag between acquisition of the ECG signals and the ability to consume the Diagnostic Quality ECG signals. A time lag considered by the art to be acceptable for clinical purposes is considered "real-time" for present purposes. "Near real-time" is a time lag that is not real-time but is as close to real-time as computing resources permit. In some embodiments, "near real time" is bounded by the length of time that might adversely delay the reaction of a caregiver to an alarm. For example, in some embodiments, a lag of 500 ms or greater may not be considered "near real time". Thus, "near real time" is as quickly as computing resources permit but not so long as delay a caregiver's reaction to an alarm.

For a second example, in a clinical care system 350, shown in FIG. 3B, the network 353 of the computing system 106 may comprise a private network 356 and a public network 359. The private network 356 may be a network in the facility 306 in which the patient 112 is located. The public network 359 may be, for instance, the Internet. The provider device 103 may transmit the Diagnostic Quality signals over the private network 356 and the public network 359 using wired or wireless connections 362, 365 to the storage 121.

The storage 121 functions as a medical records repository and is populated with a plurality of EMRs 124. The transmitted Diagnostic Quality EMR signals are saved in the respective EMR 124 for the patient 112. The caregiver 127 at the consumer device 109 is located remotely from the patient 112. In particular, in this example, the caregiver 127 is an attending physician and the remote location 115 is in the physician's office 368 located in a facility 371 other than the facility in which the patient 112 is located. The caregiver 127 may then access the respective EMR 124 for the patient 127 and once again consume the Diagnostic Quality ECG signals stored therein. The caregiver 127 may, for example, view historical cardiac activity for the patient 112 as reflected in the Diagnostic Quality ECG signals.

Those in the art having the benefit of this disclosure will appreciate still further variations and embodiments. The presently claimed subject matter admits wide variation in permutations of factors such as wired or wireless communications links, private or public networks, computing system without networking, networking protocols or not, storage or real-time consumption. Accordingly, the claimed subject matter is not limited to the embodiments illustrated herein.

Referring again momentarily to FIG 1, to further an understanding of the claimed subject matter, particular embodiments of the provider device 103 and the consumer device 109 in the illustrated embodiments. In general, it is contemplated by the present disclosure that the provider device 103 includes electronic components and/or electronic computing devices operable to receive, transmit, process, store, and/or manage patient data and information associated performing the functions of the system as described herein, which encompasses any suitable processing device adapted to perform computing tasks consistent with the execution of computer-readable instructions stored in a memory or a computer-readable recording medium.

Further, any, all, or some of the computing devices in provider device 103 and/or consumer device 109 may be adapted to execute any operating system, including Linux^{®}, UNIX^{®}, Windows Server^{®}, etc., as well as virtual machines adapted to virtualize execution of a particular operating system, including customized and proprietary operating systems. Provider device 103 and consumer device 109 may be further equipped with components to facilitate communication with other computing devices over one or more network connections, which may include connections to local and wide area networks, wireless and wired networks, public and private networks, and any other communication network enabling communication in the system.

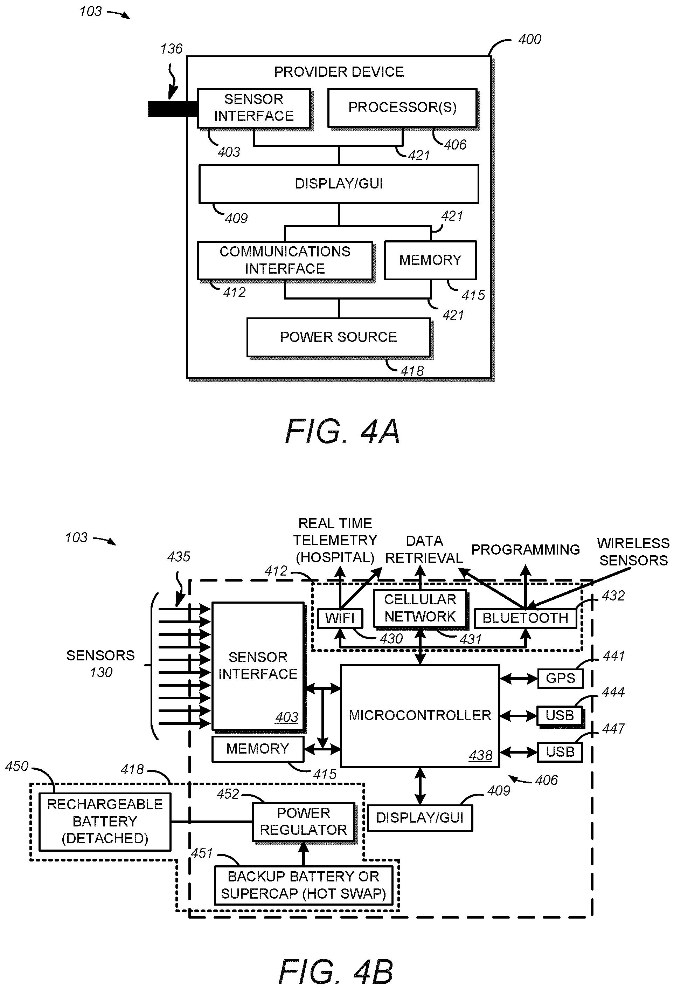

As shown in FIG. 4A, provider device 103 may be, for example, a patient monitor and, in particular, an ECG monitor 400. The provider device 103 monitors various physiological parameters of patient 112 via ECG sensors 130. The provider device 103 may include a sensor interface 403, one or more processors 406, a display/graphical user interface ("GUI") 409, a communications interface 412, a memory 415, and a power source (or power connection) 418, all communicating over an internal bus 421. The sensor interface 403 may be implemented in hardware or combination of hardware and software and is used to connect via wired and/or wireless connections to the ECG sensors 130 for gathering physiological data from the patient 112.

The data signals from the ECG sensors 130 may include, for example, sensor data related to an ECG. The one or more processors 406 may be used for controlling the general operations of the provider device 103, as well as processing sensor data received by sensor interface 403. The one or more processors 406 may be any suitable processor-based resource known to the art. They may be, but are not limited to, a central processing unit ("CPU"), a hardware microprocessor, a multi-core processor, a single core processor, a field programmable gate array ("FPGA"), a controller, a microcontroller, an application specific integrated circuit ("ASIC"), a digital signal processor ("DSP"), or other similar processing device capable of executing any type of instructions, algorithms, or software for controlling the operation and performing the functions of provider device 103. In some embodiments, the one or more processors 406 may comprise a processor chipset including, for example and without limitation, one or more co-processors.

The display/GUI 409 may be configured to display various patient data, sensor data, and hospital or patient care information, and includes a user interface implemented for allowing interaction and communication between a user and provider device 103. The display/GUI 409 may include a keyboard (not shown) and/or pointing or tracking device (not shown), as well as a display, such as a liquid crystal display ("LCD"), cathode ray tube ("CRT") display, thin film transistor ("TFT") display, light-emitting diode ("LED") display, high definition ("HD") display, or other similar display device that may include touch screen capabilities. The display/GUI 409 may provide a means for inputting instructions or information directly to the provider device 103. The patient information displayed may, for example, relate to the measured physiological parameters of the patient 112 (e.g., ECG readings).

The display/GUI 409 is optional. In some embodiments, the display/GUI 409 may be omitted where the physiological data is not going to be displayed at the patient's bedside. However, since the provider device 103 is, in this particular embodiment, an ECG monitor, the display/GUI 409 is included as data display is customary and the norm for ECG monitors.

The communications interface 412 may permit the provider device 103 to directly or indirectly (via, for example, a monitor mount) communicate with one or more computing networks and devices, workstations, consoles, computers, monitoring equipment, alert systems, and/or mobile devices (e.g., a mobile phone, tablet, or other hand-held display device). The communications interface 412 may include various network cards, interfaces, communication channels, cloud, antennas, and/or circuitry to permit wired and wireless communications with such computing networks and devices. In essence, any wireless communication protocol may be used.

Additionally, the communications interface 412 may permit direct (i.e., device-to-device) communications (e.g., messaging, signal exchange, etc.) such as from the provider device 400 through a monitor mount (not shown) to the provider device 103 using, for example, a universal serial bus ("USB") connection or other communication protocol interface. The communication interface 114 may also permit direct device-to-device connection to other devices such as to a tablet, computer, or similar electronic device; or to an external storage device or memory.

The memory 415 may be a single memory device or one or more memory devices at one or more memory locations that may include, without limitation, one or more of a random-access memory ("RAM"), a memory buffer, a hard drive, a database, an erasable programmable read only memory ("EPROM"), an electrically erasable programmable read only memory ("EEPROM"), a read only memory ("ROM"), a flash memory, hard disk, various layers of memory hierarchy, or any other non-transitory computer readable medium. The memory 415 may be on-chip or off-chip depending on the implementation of the one or more processors 406. The memory 415 may be used to store any type of instructions and patient data associated with algorithms, processes, or operations for controlling the general functions and operations of the provider device 103.

The power source 418 may include a self-contained power source such as a battery pack and/or include an interface to be powered through an electrical outlet, either directly or by way of a monitor mount. The power source 418 may also be a rechargeable battery that can be detached allowing for replacement. In the case of a rechargeable battery, a small built-in back-up battery (or super capacitor) can be provided for continuous power to be provided to the provider device 103 during battery replacement. Communication between the components of the provider device 103 in this example may be established using the internal bus 421.

The provider device 103 may be attached to one or more ECG sensors 130, first shown in FIG. 1. As previously noted, the ECG sensors 130 may be attached to the provider device 103 by the conductive leads 120 which may be, for example, cables coupled to sensor interface 403. Additionally, or alternatively, one or more ECG sensors 130 may be connected to sensor interface 403 via a wireless connection. In which case sensor interface 403 may include circuity for receiving data from and sending data to one or more devices using, for example, a WIFI^{®} connection, a cellular network connection, and/or a BLUETOOTH^{®} connection.

The data signals received from the ECG sensors 130 may be analog signals. For example, the data signals for the ECG may be input to the sensor interface 403, which can include an ECG data acquisition circuit (not shown separately in FIG. 4A). An ECG data acquisition circuit may include amplifying and filtering circuity as well as analog-to-digital ("A/D") circuity that converts the analog signal to a digital signal using amplification, filtering, and A/D conversion methods. In the event that the ECG sensor is a wireless sensor, the sensor interface 403 may receive the data signals from a wireless communication module (not shown in FIG. 4A). Thus, the sensor interface 403 is a component which may be configured to interface with the one or more ECG sensors 130 and receive sensor data therefrom.

As further described herein, the processing performed by an ECG data acquisition circuit may generate analog data waveforms or digital data waveforms that are analyzed or processed by, in this particular embodiment, one of more processors 406. However, other embodiments may use other kinds of processors disclosed above. The one or more processors 406, for example, may analyze the ECG waveforms to identify certain waveform characteristics and threshold levels indicative of conditions (abnormal and normal) of the patient 112 using one or more monitoring methods. The one or more processors 406 furthermore execute various computer-implemented methods attributable to the provider device 103 in accordance with the present disclosure.

The one or more processors 406 may be a microcontroller in some embodiments. The microcontroller may instead be, for example, a processor, an FPGA, an ASIC, a DSP, a microcontroller, or similar processing device. The microcontroller may include a memory (an on-chip memory) or use a separate memory 415 (an off-chip memory). The memory may be, for example, a RAM, a memory buffer, a hard drive, a database, an EPROM, an EEPROM, a ROM, a flash memory, a hard disk, or any other non-transitory computer readable medium. The memory 415 may store software or algorithms with executable instructions and the microcontroller may execute a set of instructions of the software or algorithms in association with executing different operations and functions of the provider device 103 such as analyzing the digital data waveforms related to the data signals from the ECG sensors 130.

To further an understanding of this particular aspect of the claimed subject matter, FIG. 4B is a schematic diagram of one particular example of the provider device 103 in FIG. 4A. The provider device 103 is, in this particular embodiment, attached to several different types of the ECG sensors 130 (including electrodes or other similar devices) known in the art for gathering physiological data related to the patient 112 (e.g., as shown on the left side of FIG. 1). The ECG sensors 130 are communicatively coupled to provider device 103 by, for example, a wired connection input to the sensor interface 403.

It is contemplated by the present disclosure that the provider device 103 can also be connected to other wireless sensors (not shown) and other devices (not shown) using the communication interface 114 of FIG. 4A. The communication interface 114 in these embodiments may include circuity for receiving data from and sending data to one or more devices using, for example, WIFI^{®} connection 430, a cellular network connection 431, and/or a BLUETOOTH^{®} connection 432. The wireless communication connections can allow, for example, patient and hospital information, alerts, and physiological data to be transmitted in real-time within a hospital wireless communications network (e.g., WIFI^{®}) as well as allow for patient and hospital information, alerts, and physiological data to be transmitted in real-time to other devices. Those in the art will appreciate that still other wireless communications protocols and/or standards may be used in addition to, or *in lieu* of, those shown in other embodiments.

It is also contemplated by the present disclosure that the communication connections established by the microcontroller 438 through the communications interface 412 permits communications over other types of wireless networks using alternate hospital wireless communications. Such alternate hospital wireless communications may include, without limitation, wireless medical telemetry service ("WMTS"), which can operate at specified frequencies (e.g., 1.4 G Hz). Other wireless communication connections can include wireless connections that operate in accordance with, but are not limited to, IEEE802.11 protocol, a Radio Frequency For Consumer Electronics ("RF4CE") protocol, ZIGBEE^{®} protocol, and/or IEEE802.15.4 protocol.

The BLUETOOTH^{®} connection 432 can also be used to provide the transfer of data to a nearby device (e.g., tablet) for review of data and/or changing of operational settings of the provider device 103. The microcontroller 438 of the provider device 103 provides a communication connection by direct wired (e.g., hard-wired) connections for transferring data using, for example, a USB connection 444 to a tablet, PC, or similar electronic device (not shown); or using, for example, a USB connection 447 to an external storage device or memory. Additionally, the microcontroller 438 includes a connection to the display/GUI 409 including a GUI for displaying patient information, physiological data or measured data, measurement schedules, alerts or alarms for the patient, clinicians and/or caregiver's information. Although the provider device 103 is described in FIG. 4B as having a single microcontroller 438, it is contemplated by the present disclosure that multiple microcontrollers can be implemented to perform the functions of the microcontroller 438.

As shown in FIG. 4B, the provider device 103 includes, in this particular embodiment, a global positioning system (GPS) or other location data system 441 that can be connected to the communication interface circuity of microcontroller 438 so that the provider device 103 can transmit to the clinician, caregiver, or other devices the location of the patient 112 at all times. Additionally, the location of the patient 112 can be used by the microcontroller 438 to determine an estimated time of arrival of the patient 112 at various locations.

For example, location data provided by the location data system 441, which may include information on a floor level, can be compared to stored information related to a hospital layout or a hospital map as well as information related to a patient's scheduled care (e.g., treatment or procedure scheduled for the patient 112 in a patient care area within the hospital). Based on the comparison results, the microcontroller 438 can determine the estimated time of arrival of the patient 112 to the patient care area within the hospital. The estimated time of arrival can be transmitted by the communication interface circuity of microcontroller 438 to, for example, the hospital wireless communications system.

Additionally, if it is determined by the microcontroller 438 that the patient 112 is not within the vicinity of the hospital wireless communications system (e.g., based on input from the location data system 441), the pertinent physiological data can be recorded and stored in the memory 415. Additionally, if the BLUETOOTH^{®} connection 432 or WIFI^{®} connection 430 are not available (e.g., out of transmission range or not operable), then the microcontroller 438 can store the physiological data in the memory 415 for later transmission when the BLUETOOTH^{®} connection or WIFI^{®} connection becomes available.

The data signals 435 from the ECG sensors 130 may be analog signals or digital signals. The data signals 435 for the ECG are input to the sensor interface 403. The sensor interface 403 may include an ECG data acquisition circuit (not otherwise shown). Both the ECG data acquisition circuit may include amplifying and filtering circuity as well as analog-to-digital (A/D) circuity that convert the analog signal to a digital signal using amplification, filtering, and A/D conversion methods known in the art.

If the received signals 435 are analog, the processing performed by the ECG data acquisition circuit of the sensor interface 403 produces digital data waveforms that are analyzed by the microcontroller 438. The one or more processors 406 shown in FIG. 4A are represented in FIG. 4B as the microcontroller 438. The microcontroller 438 includes an on-chip memory or uses the memory 415. The memory 415 stores software or algorithms with executable instructions and the microcontroller 438 can execute a set of instructions of the software or algorithms in association with executing different operations and functions of the provider device 103 such as analyzing the digital data waveforms related to the data signals from the ECG sensors 130. More particularly, the microcontroller 438, for example, executes instructions residing on the memory 415 to perform the programmed methods attributable to the provider device 103 as described herein.

The power source 418 shown in FIG. 4A is represented by elements 450-452 in FIG. 4B. As shown in FIG. 4B, the power can be supplied using a rechargeable battery 450 that can be detached allowing for replacement. The rechargeable battery 450 may be, for example, a rechargeable lithium-ion battery. Additionally, a small built-in backup battery 451 (or supercapacitor) is provided for continuous power to the provider device 103 during battery replacement. A power regulator or regulation circuit 452 is provided between the rechargeable battery 450 and small backup battery 451 to control which battery provides power to the provider device 103.

It is also contemplated by the present disclosure that the power regulator 452 can include a self-contained power source such as a battery pack and/or include an interface to be powered through an electrical outlet (either directly or by way of a monitor mount). Communication between the components of the provider device 103 can be established using an internal bus similar to the internal bus 421 discussed with reference to FIG. 4A.

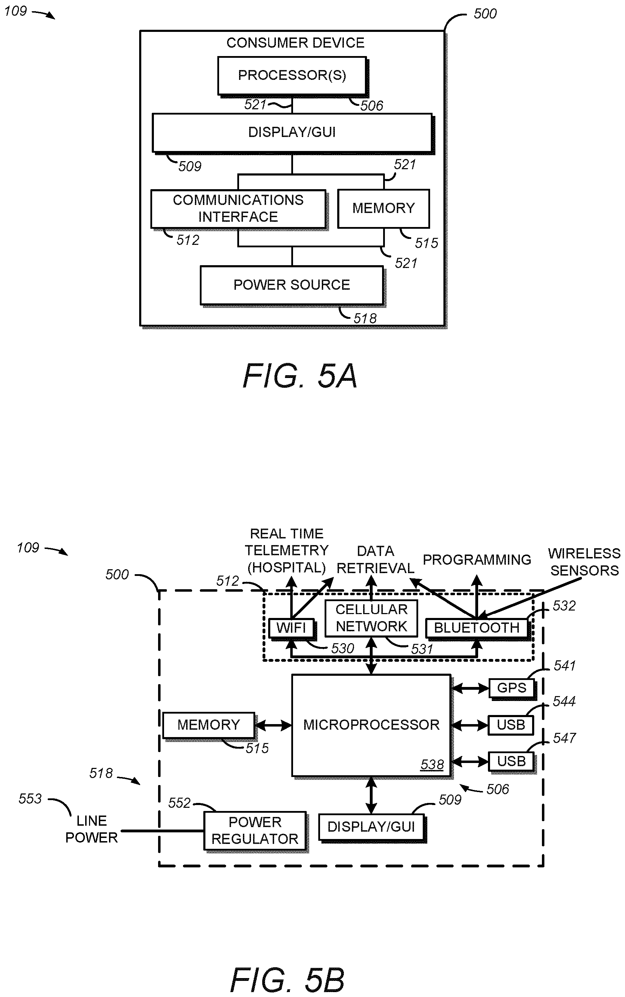

Turning now to FIG. 5A, the consumer device 109 may be, for example, a computing apparatus of some type as discussed above. The consumer device 109 may include one or more processors 506, a display/graphical user interface ("GUI") 509, a communications interface 512, a memory 515, and a power source (or power connection) 518, all communicating over an internal bus 521. The display/GUI 509 is optional and may be omitted in some embodiments as described further below.

The one or more processors 506 may be any suitable processor-based resource. They may be, but are not limited to, a central processing unit ("CPU"), a hardware microprocessor, a multi-core processor, a single core processor, a field programmable gate array ("FPGA"), a controller, a microcontroller, an application specific integrated circuit ("ASIC"), a digital signal processor ("DSP"), or other similar processing device capable of executing any type of instructions, algorithms, or software for controlling the operation and performing the functions of consumer device 109. In some embodiments, the one or more processors 506 may comprise a processor chipset including, for example and without limitation, one or more co-processors.

The display/GUI 509 may be configured to display various patient data, sensor data, and hospital or patient care information, and includes a user interface implemented for allowing interaction and communication between a user and consumer device 109. The display/GUI 509 may include a keyboard (not shown) and/or pointing or tracking device (not shown), as well as a display, such as a liquid crystal display ("LCD"), cathode ray tube ("CRT") display, thin film transistor ("TFT") display, light-emitting diode ("LED") display, high definition ("HD") display, or other similar display device that may include touch screen capabilities. The display/GUI 509 may provide a means for inputting instructions or information directly to the consumer device 109. The patient information displayed may, for example, relate to the measured physiological parameters of the patient 112 (e.g., ECG readings).

The communications interface 512 may permit the consumer device 109 to directly or indirectly (via, for example, a monitor mount) communicate with one or more computing networks and devices, workstations, consoles, computers, monitoring equipment, alert systems, and/or mobile devices (e.g., a mobile phone, tablet, or other hand-held display device). The communications interface 512 may include various network cards, interfaces, communication channels, cloud, antennas, and/or circuitry to permit wired and wireless communications with such computing networks and devices. In essence, any wireless communication protocol may be used.

Additionally, the communications interface 512 may permit direct (i.e., device-to-device) communications (e.g., messaging, signal exchange, etc.) such as from the provider device 103 through a monitor mount (not shown) to the consumer device 109 using, for example, a universal serial bus ("USB") connection or other communication protocol interface. The communication interface 114 may also permit direct device-to-device connection to other devices such as to a tablet, computer, or similar electronic device; or to an external storage device or memory.

The memory 515 may be a single memory device or one or more memory devices at one or more memory locations that may include, without limitation, one or more of a random-access memory ("RAM"), a memory buffer, a hard drive, a database, an erasable programmable read only memory ("EPROM"), an electrically erasable programmable read only memory ("EEPROM"), a read only memory ("ROM"), a flash memory, hard disk, various layers of memory hierarchy, or any other non-transitory computer readable medium. The memory 515 may be on-chip or off-chip depending on the implementation of the one or more processors 506. The memory 515 may be used to store any type of instructions and patient data associated with algorithms, processes, or operations for controlling the general functions and operations of the consumer device 109.

The power source 518 may include a self-contained power source such as a battery pack and/or include an interface to be powered through an electrical outlet, either directly or by way of a monitor mount. The power source 518 may also be a rechargeable battery that can be detached allowing for replacement. In the case of a rechargeable battery, a small built-in back-up battery (or super capacitor) can be provided for continuous power to be provided to the consumer device 109 during battery replacement. Communication between the components of the consumer device 109 in this example may be established using the internal bus 521.

The data handling, processing, and analysis attributed to the consumer device 500 may be performed by, in this particular embodiment, one of more processors 506. The one or more processors 506 furthermore execute various computer-implemented methods attributable to the consumer device 109 in accordance with the present disclosure. For example, the one or more processors 506 may include some kind of rendering for display at Diagnostic Quality or analyses such as 12-lead reports or S-T segment analysis.

To further an understanding of this particular aspect of the claimed subject matter, FIG. 5B is a schematic diagram of one particular example of the consumer device 500 in FIG. 5A. The consumer device 500 includes the one or more processors 506, a display/graphical user interface ("GUI") 509, a communications interface 512, a memory 515, and a power source (or power connection) 518, all communicating over an internal bus 521 as discussed relative to FIG. 5A. FIG. 5B, however, illustrates particular implementations of some of those components in this embodiment.

The one or more processors 506 may be a microprocessor 538 in some embodiments. The microprocessor 538 may alternatively be, for example, a processor, an FPGA, an ASIC, a DSP, a microcontroller, or similar processing device. The microprocessor 538 may include a memory (an on-chip memory) or use a separate memory 515 (an off-chip memory). The memory may be, for example, a RAM, a memory buffer, a hard drive, a database, an EPROM, an EEPROM, a ROM, a flash memory, a hard disk, or any other non-transitory computer readable medium. The memory 515 may store software or algorithms with executable instructions and the microprocessor 538 may execute a set of instructions of the software or algorithms in association with executing different operations and functions of the consumer device 109 such as analyzing the digital data waveforms related to the data signals from the ECG sensors 130.

It is contemplated by the present disclosure that the consumer device 109 can also be connected to other wireless sensors (not shown) and other devices (not shown) using the communication interface 512 of FIG. 5A. The communication interface 512 in these embodiments may include circuity for receiving data from and sending data to one or more devices using, for example, WIFI^{®} connection 530, a cellular network connection 531, and/or a BLUETOOTH^{®} connection 532. The wireless communication connections can allow, for example, patient and hospital information, alerts, and physiological data to be transmitted in real-time within a hospital wireless communications network (e.g., WIFI^{®}) as well as allow for patient and hospital information, alerts, and physiological data to be transmitted in real-time to other devices. Those in the art will appreciate that still other wireless communications protocols and/or standards may be used in addition to, or *in lieu* of, those shown in other embodiments.

It is also contemplated by the present disclosure that the communication connections established by the microprocessor 538 through the communications interface 512 permits communications over other types of wireless networks using alternate hospital wireless communications. Such alternate hospital wireless communications may include, without limitation, wireless medical telemetry service ("WMTS"), which can operate at specified frequencies (e.g., 1.4 G Hz). Other wireless communication connections can include wireless connections that operate in accordance with, but are not limited to, IEEE802.11 protocol, a Radio Frequency For Consumer Electronics ("RF4CE") protocol, ZIGBEE^{®} protocol, and/or IEEE802.15.4 protocol.

The BLUETOOTH^{®} connection 532 can also be used to provide the transfer of data to a nearby device (e.g., tablet) for review of data and/or changing of operational settings of the consumer device 109. The microprocessor 538 of the consumer device 109 provides a communication connection by direct wired (e.g., hard-wired) connections for transferring data using, for example, a USB connection 544 to a tablet, a personal computer ("PC"), or similar electronic device (not shown); or using, for example, a USB connection 547 to an external storage device or memory. Additionally, the microprocessor 538 includes a connection to the display/GUI 509 including a GUI for displaying patient information, physiological data or measured data, measurement schedules, alerts or alarms for the patient, clinicians and/or caregiver's information. Although the consumer device 109 is described in FIG. 5B as having a single microprocessor 538, it is contemplated by the present disclosure that multiple microprocessors can be implemented to perform the functions of the microprocessor 538.

As shown in FIG. 5B, the consumer device 109 includes, in this particular embodiment, a global positioning system (GPS) or other location data system 541 that can be connected to the communication interface circuity of microprocessor 538 so that the consumer device 109 can transmit to the clinician, caregiver, or other devices the location of the patient 112 (shown in FIG. 1). Additionally, the location of the patient 112 can be used by the microprocessor 538 to determine an estimated time of arrival of the patient 112 at various locations.

For example, location data provided by the location data system 541, which may include information on a floor level, can be compared to stored information related to a hospital layout or a hospital map as well as information related to a patient's scheduled care (e.g., treatment or procedure scheduled for the patient 112 in a patient care area within the hospital). Based on the comparison results, the microprocessor 538 can determine the estimated time of arrival of the patient 112 to the patient care area (not shown) within the hospital. The estimated time of arrival can be transmitted by the communication interface circuity of microprocessor 538 to, for example, the hospital wireless communications system.

Additionally, if it is determined by the microprocessor 538 that the patient 112 is not within the vicinity of the hospital wireless communications system (e.g., based on input from the location data system 541), the pertinent physiological data can be recorded and stored in the memory 515. Additionally, if the BLUETOOTH^{®} connection 532 or WIFI^{®} connection 530 are not available (e.g., out of transmission range or not operable), then the microprocessor 538 can store the physiological data in the memory 515 for later transmission when the BLUETOOTH^{®} connection or WIFI^{®} connection becomes available.

The one or more processors 506 shown in FIG. 5A are represented in FIG. 5B as the microprocessor 538. The microprocessor 538 includes an on-chip memory or uses the memory 515. The memory 515 stores software or algorithms with executable instructions and the microprocessor 538 can execute a set of instructions of the software or algorithms in association with executing different operations and functions of the consumer device 109 such as analyzing the digital data waveforms related to the data signals from the provider device 103. More particularly, the microprocessor 538, for example, executes instructions residing on the memory 515 to perform the programmed methods attributable to the consumer device 109 as described herein.

The power source 518 shown in FIG. 5A is represented by the power regulator 552 and line power 553 in FIG. 5B. Some embodiments may employ one or more backup batteries such as the batteries discussed relative to FIG. 4B in the even the line power 553 fails. The power regulator or regulation circuit 552 is provided between the rechargeable battery 550 and small backup battery 551 to control which battery provides power to the consumer device 109. It is also contemplated by the present disclosure that the power regulator 552 can include a self-contained power source such as a battery pack and/or include an interface to be powered through an electrical outlet (either directly or by way of a monitor mount). Communication between the components of the consumer device 109 can be established using an internal bus similar to the internal bus 521 discussed with reference to FIG. 5A.

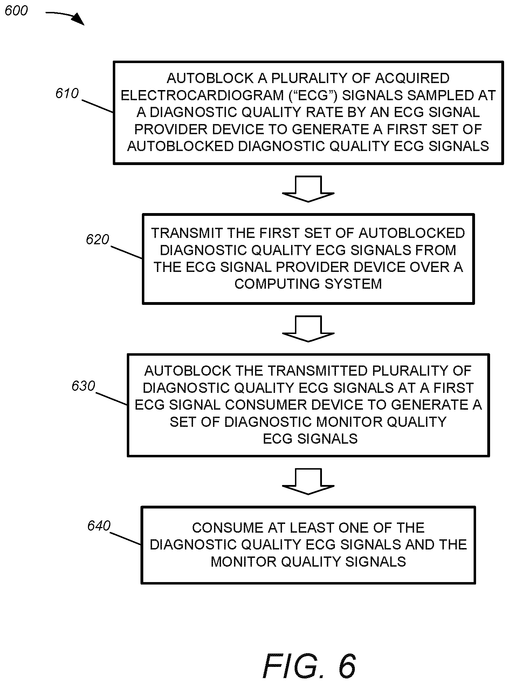

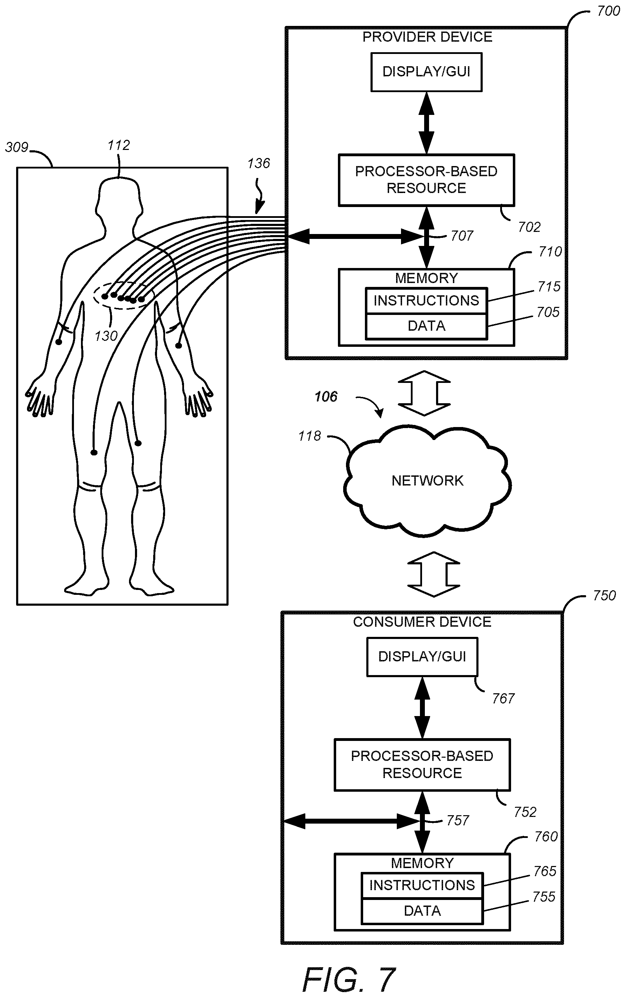

FIG. 6 illustrates a computer-implemented method 600 for use in monitoring the physical condition of a patient in a clinical care system. FIG. 7 schematically depicts one particular implementation of a provider device 700 and one particular implementation of a consumer device 750. The depictions of the provider device 700 and the consumer device 750 are simplified from what has been previously shown and discussed so as not to obscure those aspects of the claimed subject matter now under discussion. As illustrated in FIG. 7, a processor-based resource 702 communicates with a memory 710 over an internal bus system 707 in the provider device 700. A processor-based resource 752 communicates with a memory 710 over an internal bus system 757 in the consumer device 750.

The processor-based resources 702, 752 may be, for example, one of the one or more processors 406, 506 shown in FIGs. 4A, 5A, including the microcontrollers 438, 538 shown in FIGs. 4B, 5B. The processor-based resources 702, 752 operate under programmed control of the instructions 715, 765, respectively. The processor-based resources 702, 752 therefore communicate with the memories 710, 760, respectively, over the bus systems 707, 757, respectively, to, among other things, execute the instructions 715, 765 stored thereon.

The execution of the instructions may cause the processor-based resource 702 to perform certain tasks such as some portions of the method 600 outlined in FIG. 6. However, depending on the environment, the processor-based resources 702, 752 may also perform other tasks such as pushing data to other computing resources, transmitting alarms, *etc.* Thus, in this sense, the processor-based resources 702, 752 as well as the provider device 700 and consumer device 750 as a whole, may be considered to be "programmed" or "configured" to perform certain functions as described herein.

Referring now to FIGs. 6-7 collectively, the method 600 presumes that the ECG signals have previously been acquired from the patient 112 using the lead assemblies 136. The processor-based resource 702 executing the instructions 715 may process the ECG signals in real-time or may access them in near real-time as the data 705 buffered in the memory 710. The ECG signals may be acquired as discussed above relative to the method 200 in FIG. 2.

The method 600 begins by autoblocking (at 610) a plurality of acquired electrocardiogram ("ECG") signals sampled at a Diagnostic Quality rate by a provider device to generate a first set of autoblocked Diagnostic Quality ECG signals. In the illustrated embodiment, the Diagnostic Quality rate is a minimum of 300 samples per second ("sps"). This is the minimum Nyquist sample rate for an acquired ECG signal of 150 Hz. However, to provide an adequate margin over the minimum Nyquist sample rate of 300 sps, the illustrated embodiments employ a Diagnostic Quality sample rate of 500 sps. This will produce autoblocked Diagnostic Quality ECG signals between .05-150 Hz.

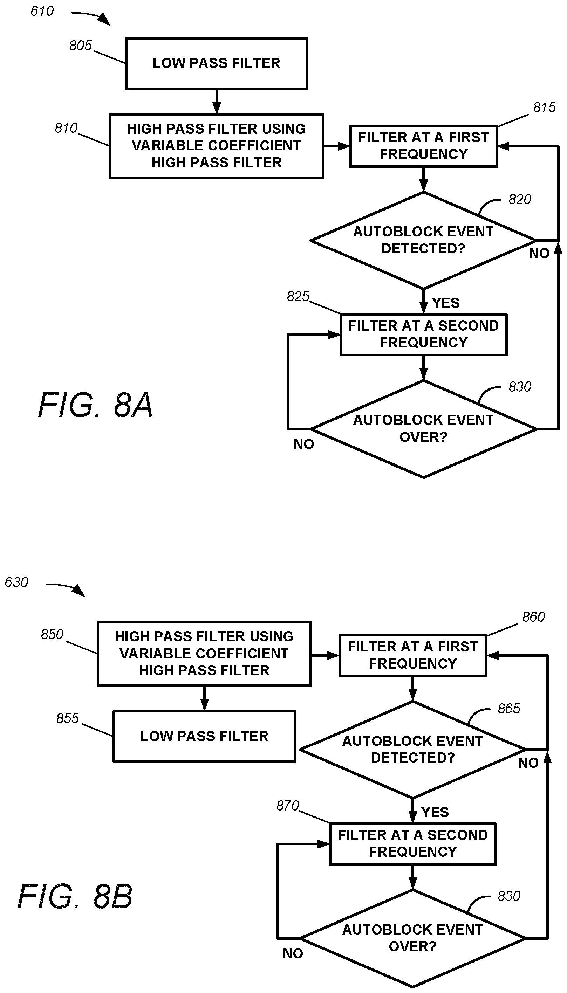

The autoblocking (at 610) technique of the illustrated embodiment is illustrated in FIG. 8A. The autoblocking (at 610) begins by low pass filtering (at 805) the acquired ECG signals. The low pass filter may be, for example, an Infinite Impulse response ("IIR") filter. In the illustrated embodiment, the low pass filtering (at 805) includes a 0-524 Hz passband.

Next, the autoblocking (at 610) high pass filters (at 810) the low pass filtered ECG signals using a variable coefficient high pass filter. The high pass filtering (at 810) first employs the variable coefficient high pass filter filtering (at 815) at a first frequency until an autoblocking event is detected (at 820). Upon detecting the autoblocking event (at 820), the variable coefficient high pass filter filters (at 825) at a second frequency. Once the autoblocking event concludes (at 830), the variable coefficient high pass filter again filters at the first frequency (at 815). In the illustrated embodiment, the passband for the low pass filtering is 0-524 Hz, the first frequency is .05 Hz, and the second frequency is 5 Hz.

Returning now to FIGs. 6-7 collectively, the autoblocking (at 610) by the provider device 700 generates a first set of autoblocked Diagnostic Quality ECG signals. The provider device 700 then transmits (at 620) the first set of autoblocked Diagnostic Quality ECG signals from the provider device 700 over a computing system 106. The claimed subject matter admits wide variation in the implementation of the computing system 106 as is discussed relative to FIGs. 1 and 3A-3B.

The transmission occurs in real-time if computing resources permit. If computing resources are insufficient for real-time transmission, the autoblocked Diagnostic Quality ECG signals may be buffered as data 705 in the memory 710. Either way, as mentioned above and as will be discussed more fully below, the autoblocked Diagnostic Quality ECG signals may be packetized and transmitted. Alternatively, the autoblocked Diagnostic Quality ECG signals may be transmitted point by point. However, point by point transmission can undesirably increase processing and, so, some embodiments may transmit the Diagnostic Quality ECG signals point by point at the packet boundary. Still other embodiment may use any other suitable technique known to the art.

The transmitted Diagnostic Quality ECG signals are received by the consumer device 750 from the provider device 700. The processor-based resource 752 executing the instructions 765 may process the ECG signals in real-time or may access them in near real-time as the data 755 buffered in the memory 760. Either way, the consumer device 750 autoblocks (at 630) the transmitted set of Diagnostic Quality ECG signals at a first ECG signal consumer device to generate a set of Monitor Quality ECG signals.

The autoblocking technique (at 630) is illustrated in FIG. 8B. The autoblocking technique (at 630) begins with high pass filtering (at 850) followed by low pass filtering (at 855). The high pass filtering (at 850) begins by applying a variable coefficient high pass filter that filters (at 860) at a first frequency until an autoblocking event is detected (at 865). Upon detecting (at 865) the autoblocking event, the variable coefficient high pass filter filters (at 870) at a second frequency. Once the autoblocking event concludes (at 875), the variable coefficient high pass filter again filters (at 860) at the first frequency. In the illustrated embodiment, the passband for the low pass filtering is 40 Hz or 17 Hz, the first frequency is 0.67 Hz, and the second frequency is 5 Hz.

Referring again to FIGs. 6-7 collectively, the method 600 concludes with the consumer device 750 consuming (at 640) at least one of the Diagnostic Quality ECG signals and the Monitor Quality signals. As discussed above, "consuming" means to use the ECG signals in some useful and practical application in a clinical care context. The consumption of the data can include using the received ECG signals upon receipt or storing them for later review. The manner in which the ECG signals are consumed will be implementation specific. In some embodiments, as discussed above, the second set of Diagnostic Quality ECG signals may be consumed by displaying them on the display/GUI 767 of the consumer device 750. In other embodiments, the second set of Diagnostic Quality ECG signals may be consumed by performing analyses such as 12-lead reports or S-T segment analysis. Those in the art having the benefit of this disclosure will appreciate still other ways in which the ECG signals may be consumed.

In some embodiments, the consumer device 750 may filter the second set of Diagnostic Quality signals to generate a set of Monitor Quality signals. The variable coefficient high pass filter of the autoblocking technique (at 630) employed by the consumer device 750 filters down from Diagnostic Quality to Monitor Quality. Note, however, that reversing the process to achieve Diagnostic Quality from Monitor Quality involves interpolation that can introduce unwanted artifacts. The variable coefficient HP filter will achieve lower end of Monitor Quality bandwidth (0.67Hz); and the higher end of BW (40Hz) will need 2:1 decimation with a low pass filter. The low pass filter is not a variable coefficient low pass filter.

Note that although the autoblocking (at 610, 630) is performed in real-time or near real-time, the consumption (at 640) is not so limited. To be sure, there will be embodiments in which the consumption (at 640) occurs in real-time or near real-time. However, as discussed relative to the embodiment of FIG. 1, the ECG signals may be stored in a medical records repository on the storage 121. Such stored ECG signals may later be accessed by, for example, by a caregiver 127 days, weeks, or months, afterward.

Note that in the illustrated embodiments the autoblocking is performed in software that may be embodied in, for example, the instructions 715, 765 and executed by the processor-based resources 702, 752. This software may be, for example, firmware in some embodiments. However, some embodiments may choose to instead perform the autoblocking in hardware or in some combination of hardware and software. Accordingly, the claimed subject matter is not necessarily limited to software-implemented autoblocking in all embodiments.

Referring again to FIG. 1, as mentioned above, some embodiments may transmit the autoblocked Diagnostic Quality ECG signals from the provider device 103 to the consumer device 109 in discrete "packets". Packets of information may be "packetized" in accordance with any number of networking protocols. One such networking protocol is TCP/IP, which is the networking protocol used in the embodiment of FIG. 1. However, any suitable networking protocol known to the art may be used.

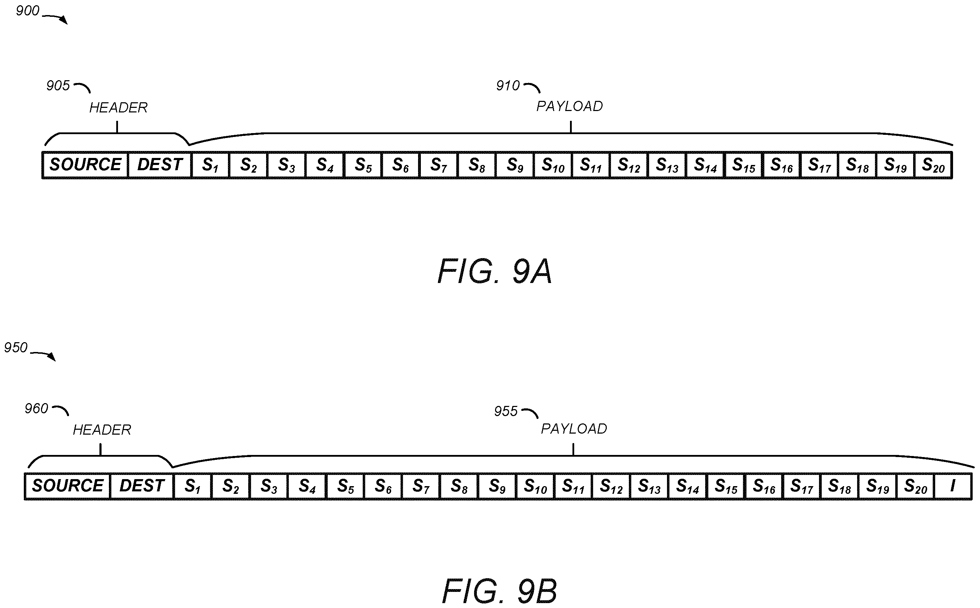

The composition and formatting of each packet will depend on the protocol employed. For example, TCP and IP each have different formatting specifications. However, to the extent they can be generalized, and as shown in FIG. 9A, a packet 900 typically comprises a header 905 and a payload 910. The headers 905 includes administrative information such as the source location, the destination location, various flags, etc. In FIG. 9A, the header 905 is shown including the source and destination as representative header information, but those skilled in the art having the benefit of this disclosure will appreciate that the header 905 will typically include a great deal more information.

Table 1 and Table 2 list the packet composition and formatting for TCP and IP packets. In each of Table 1 and Table 2, each field except the payload may be considered "header" information. The tables include field name, number of bits, and a description. Those in the art having the benefit of this disclosure will appreciate that there may be variations from what is represented in Table 1 and Table 2, particularly when alternative protocols are employed. Additional information is available from the respective standards for the networking protocols.

**TABLE 1. TCP PACKET COMPOSITION & FORMATTING**

| Field Name | # of Bits | Description |
|---|---|---|
| Source Port | 16 | Source application's port number and helps in determining the application where the data delivery is planned. |
| Destination Port | 16 | Port number of the transmitting application and helps to send the data to the appropriate application. |
| Sequence # | 32 | Sequence of the packet payload in the packetized communication used to ensure that the data is received in proper order by |
| | | ordered segmenting and reassembling them at the receiving end. |
| Acknowledgement # | 32 | The upcoming sequence number that acknowledges the feedback up to that point. |
| Data Offset | 4 | indicates the starting point of the TCP data payload also storing the size of the TCP header. |
| Control Flags | 9 | Control flags used to regulate communication and indicate conditions such as reset, termination, acknowledgement, and synchronization. |
| Window Size | 16 | Indicates the size of the sender's receive window. |
| Checksum | 16 | Used to determine whether the header was damaged during transport. |
| Urgent Pointer | 16 | Points to the packet's first byte of urgent data. |
| Options | Variable | Represents selected predetermined TCP options. |
| Data Payload | Undefined | The data being transmitted. |

**TABLE 2. IP PACKET COMPOSITION & FORMATTING**

| Field | # of Bits | Description |
|---|---|---|
| Version | 4 | Indication of which version is being used. |
| Header Length | 4 | Indicates how many 32-bit words are present in the IP header. |
| Type of Service | 8 | Information about how the quality of the service is being delivered. |
| Total Length | 16 | The total length of the IP packet is stored in bytes. |
| Identification | 16 | Specific identity for the particular packet. |
| Flags | 3 | Control flags for packet fragmentation and reassembly. |
| Fragment Offset | 13 | Identifies the location of a packet inside the original message in the number of data bytes ahead of the fragment. |
| Time to Live | 8 | Specifies the total lifetime of the packet in the internet system in terms of number of hops the packet can make before being terminated. |
| Protocol | 8 | Specifies the type of transport layer protocol, such as TCP, UDP, or ICMP, to which the IP packet will be routed. |
| Header Checksum | 16 | Error checking value for identifying errors in the header. |
| Source IP Address | 16 | Sender's 32 bit address. |
| Destination IP Address | 32 | Destinations IP address. |
| Options | Variable | Indicates options and parameters for conditions such security, record route, time stamp, etc. |

Referring again to FIG. 9A, the payload 910 includes, in this particular embodiment, twenty samples S₁-S₂₀ of the autoblocked ECG Diagnostic Quality signals transmitted by the provider device. At a sampling rate of 500 sps, the twenty samples represent 40 ms of raw ECG signal. Each sample comprises one or more bits or bytes, depending on the implementation, representing the value of the sample. Note that alternative embodiments may packetize more or fewer samples in a packet so long as the packet size is permitted by the applicable protocol.

In some embodiments, the transmitted Diagnostic Quality signals may be accompanied by an "autoblock indicator". The autoblock indicator may indicate when one or more samples in the transmitted ECG signals have been autoblocked. This information can be displayed when the whenever a caregiver is consuming the ECG signals, whether Monitor Quality or Diagnostic Quality. The autoblock indicator may be used to indicate to a caregiver when a portion of a displayed waveform has resulted from an autoblocking event. For example, during the display of the Diagnostic Quality signal, the waveform may ordinarily be displayed in a first color and then displayed in a second color when the waveform results from an autoblocking event. Those in the art having the benefit of this disclosure may realize other ways in which this information may be conveyed to the caregiver during display.

For example, FIG. 9B illustrates a generalized packet 950 in which the payload 955 includes an autoblock indicator bit *I*, indicating that the samples *S₁-S₂₀* have been subjected to autoblock by the provider device. The autoblock indicator will more typically be located in the header 960 in most embodiments, for example as a control flag or a status bit in the header. However, the claimed subject matter is amenable to both approaches in packetized transmission embodiments.

Some embodiments may eschew packetized transmission for point by point transmission as was mentioned above. In such embodiments, the autoblock indicators may be transmitted as a bit stream separate and apart from the transmitted ECG Diagnostic Quality ECG signals. Or, alternatively, the autoblock indicator bit stream may be multiplexed with the Diagnostic Quality ECG signals for transmission. Those in the art having the benefit of this disclosure may realize still other approaches for transmitting the autoblock indicator in point by point transmission embodiments.

Returning to FIGs. 1-2 and 6, the transmitted Diagnostic Quality ECG signals are received by the consumer device 109. The transmitted Diagnostic Quality signals may be transmitted in parallel to the storage 121, which is also considered a consumer device in this embodiment. The storage 121 is serving as an electronic records repository populated by a plurality of electronic medical records ("EMRs") 124. Or, the transmitted Diagnostic Quality signals be stored from the consumer device 109.

Either way, the Diagnostic Quality ECG signals are transmitted to the consumer device 109 either directly or indirectly. Directly, in this context, means transmission is directed to the consumer device 109 without being stored or archived in some kind of long term storage such as an electronic records repository. Indirectly, in this context, means that the Diagnostic Quality signals are first stored or archived in a long term storage by the consumer device 109. Again, note that the act of storing the Diagnostic Quality signals is a "consumption" also and that the long term storage consequently is also considered a consumer device.

The transmitted and received Diagnostic Quality signals are then autoblocked (at 630) to generate the Monitor Quality signals. The autoblocking (at 630) may be performed at the time of consumption or at the time of receipt if those two events are not concurrent. The resolution step down from Diagnostic Quality to Monitor Quality is a function of the variable high pass filter used in the autoblocking.

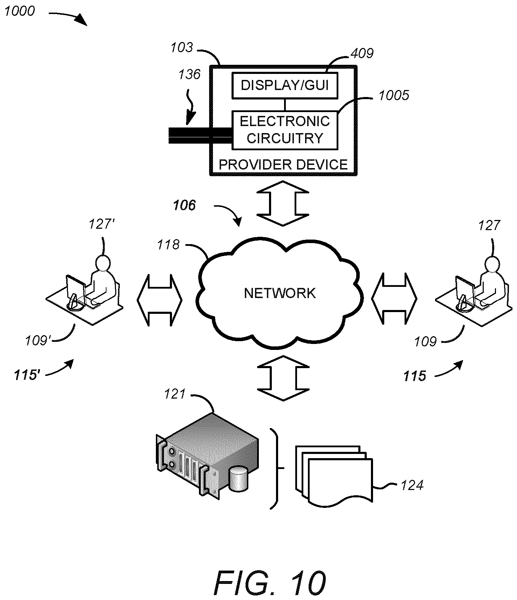

FIG. 10 illustrates a particular embodiment of a clinical care system in which technique described herein may be implemented in one or more embodiments. The clinical care system 1000 is a variation on embodiments previously presented above, with like parts bearing like numbers. One aspect of the variation is a second consumer device 109' operated by a second caregiver 127' and a second remote location 109'. Another variation is that various aspects of the provider device 103 previously shown in FIG. 4A, such as the sensor interface 403, processor(s) 406, communications interface 412, and memory 415, are represented collectively as the electronic circuitry 1005.

In the embodiment of FIG. 10, the provider device 103 is an ECG monitor located at the bedside of the patient (not shown) to acquire data over the lead assemblies 136. The first consumer device 109 is located at the first remote location 115, which is a nursing station in this embodiment, and the located in a different building than are the provider device 103 and the first remote location 109. The caregiver 127' is an attending physician that may be monitoring and/or evaluating the caregiver 127 is a nurse. The second remote location 109' is a doctor's office patient's condition.

In one scenario, the provider device 103 receives the data over the lead assemblies 136 through the electronic circuitry 1005. The data is then processed by the electronic circuitry 1005 as described above. The data may be displayed on the display/GUI 409 of the provider device 103 at either Diagnostic Quality or at Monitor Quality. In the illustrated embodiments, the data is displayed by the provider device 103 using autoblocked Diagnostic Quality signals produced by the electronic circuitry.

Where the data is displayed in Monitor Quality, the electronic circuitry 1005 may process the data in parallel to obtain the Monitor Quality signals while processing the data to obtain the Diagnostic Quality signals as described above. As noted above, the quality of resultant signals is a function of the variable high pass filter of the autoblocking implemented, in the disclosed embodiments, in the software of the electronic circuitry 1005. Thus, where the data is displayed by the provider device 103in Monitor Quality, the data is processed in parallel. In one branch, the data is autoblocked using a first variable high pass filter to yield Monitor Quality signals and, in a second branch, is autoblocked using a second variable high pass filter to yield Diagnostic Quality signals.

The Diagnostic Quality signals are then transmitted to the first remote location 115 and the second remote location 115' as described above. The Diagnostic Quality signal are then processed at the first and second consumer devices 109, 109' as described above, including autoblocking as described, to produce Monitor Quality signals. The Monitor Quality signals can then be displayed on the displays (not otherwise shown) of the first and second consumer devices 109, 109'. The first and second caregivers 127, 127' can therefore monitor the patient's cardiac activity without being at the patient's bedside or even in the same room as the provider device 103.

The caregiver 127' may wish to achieve a different perspective on the patient's cardiac condition by performing additional, or different, analyses of the Digital Quality signals. For example, the caregiver 127', a physician, may wish to see a 12-lead report or a S-T segment analysis for the patient. The caregiver 127' may access Diagnostic Quality signals from the EMRs 124 of the electronic records repository to conduct such studies.

The scenario presented in FIG. 10 illustrates how the presently disclosed technique may help reduce congestion and traffic on the computing system of the clinical care system. Because the Monitor Quality signals consumed by the consumer devices are derived from the Diagnostic Quality signals, only the Diagnostic Quality signals need be transmitted over the computing system. (Note, however, that in some embodiments the Monitor Quality signals may nevertheless be transmitted over the computing network should this be desired.) Thus, the bandwidth of the computing system need only accommodate the Diagnostic Quality signals. Furthermore, autoblocking on both the provider and consumer sides of the clinical care system prevents, or at least inhibits, the introduction of undesirable artifacts in the transmitted Diagnostic Quality signals that would otherwise be present due to the transmission.

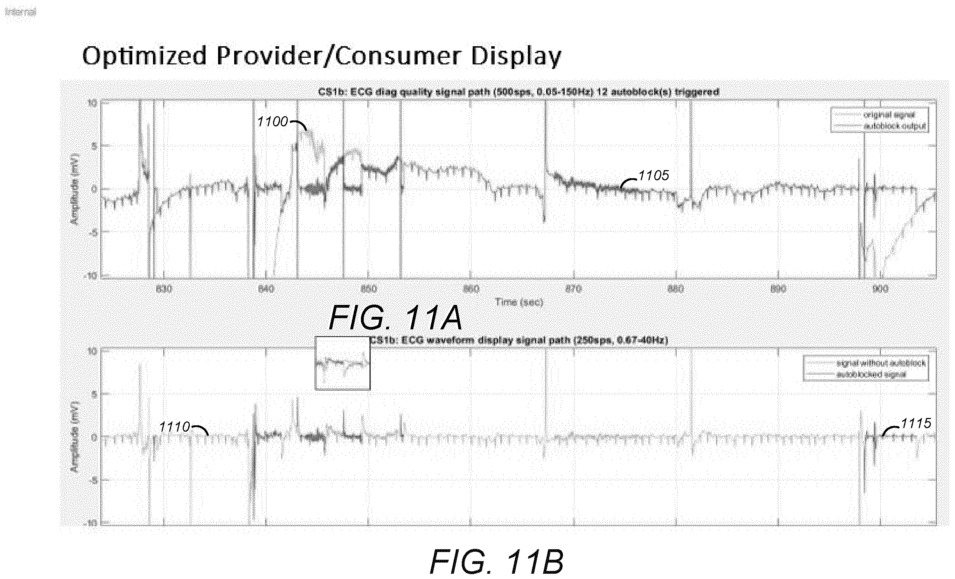

FIG. 11A-FIG 11B illustrate the efficacy of the technique disclosed herein. Figure 11A illustrates an original, Diagnostic Quality (e.g., 500 sps, 0.05-150 Hz) ECG signal 1100 (green) and an autoblocked version 1105 (red) of that original signal 1100. The color coding is used to indicate when autoblocking is occurring to inform the caregiver and for risk mitigation. Both the provider device and the consumer device use the Diagnostic Quality ECG signal for waveform display. The term "original" in this context means the acquired ECG signals prior to being autoblocked. Generation of the autoblocked version 1105 included twelve (12) autoblock events.

FIG. 11B illustrates a second original, Diagnostic Quality (e.g., 250 sps, 0.67-40 Hz) signal 1110 (green) and an autoblocked version 1115 (red). Again, the color coding is used to indicate when autoblocking is occurring to inform the caregiver and for risk mitigation. Note that, in both cases, adding autoblocking to the consumer side removes baseline artifacts that would otherwise be caused by the transmission.

One principle application of the presently disclosed technique is to facilitate 12 lead analysis on historical data, although other applications are available. These applications are practical applications of the technique within the clinical care environment. Furthermore, in order to be able to do that, higher fidelity data-e.g., Diagnostic Quality-is needed on the system. The easy way to solve the problem is to send both 250 SPS (e.g., Monitor Quality) data and 500 SPS (e.g., Diagnostic Quality) data on the wire continuously. However, as discussed above, this creates problems associated with traffic volume and congestion on the computing system. The presently disclosed technique reduces traffic and congestion on the network by combining both streams of data. The presently closed technique thereby also improves the performance and functionality of the computing system as a whole.

Accordingly, in a the first embodiment, a computer-implemented method comprises: autoblocking a plurality of acquired electrocardiogram ("ECG") signals sampled at a Diagnostic Quality rate by an ECG signal provider device to generate a first set of autoblocked Diagnostic Quality ECG signals; transmitting the first set of autoblocked Diagnostic Quality ECG signals from the ECG signal provider device over a computing system; autoblocking the transmitted first set of autoblocked Diagnostic Quality ECG signals at a first ECG signal consumer device to generate a second set of Diagnostic Quality ECG signals; and consuming the second set of twice autoblocked Diagnostic Quality ECG signals.

In a second embodiment, the computer-implemented method of the first embodiment, further comprises: autoblocking the transmitted plurality of Diagnostic Quality ECG signals at one of a plurality of ECG signal consumer devices, the plurality of ECG signal consumer devices including the first ECG signal consumer device, to generate a set of Monitor Quality ECG signals; and consuming the set of Monitor Quality ECG signals.

In a third embodiment, in the computer-implemented method of the first embodiment, transmitting the first set of autoblocked Diagnostic Quality ECG signals includes transmitting an autoblocking indicator.

In a fourth embodiment, in the computer-implemented method of the third embodiment, transmitting the first set of autoblocked Diagnostic Quality ECG signals and the autoblocking indicator includes transmitting the first set of autoblocked Diagnostic Quality ECG signals and the autoblocking indicator point by point.

In a fifth embodiment, in the computer-implemented method of the third embodiment, transmitting the first set of autoblocked Diagnostic Quality ECG signals and the autoblocking indicator includes packetizing and transmitting the first set of autoblocked Diagnostic Quality ECG signals and the autoblocking indicator point by point on the packet boundary.

In a sixth embodiment, in the computer-implemented method of the third embodiment, consuming the second set of autoblocked Diagnostic Quality ECG signals includes performing a 12-lead reports or a S-T segment analysis.

In the seventh embodiment, in the computer-implemented method of the third embodiment, consuming the second set of autoblocked Diagnostic Quality ECG signals includes displaying the Monitor Quality signals.

In an eighth embodiment, the computer-implemented method of the first embodiment further comprises: autoblocking the transmitted first set of autoblocked Diagnostic Quality ECG signals at a second ECG signal consumer device to generate a third set of Diagnostic Quality ECG signals; and consuming the third set of autoblocked Diagnostic Quality ECG signals.

In a ninth embodiment, in the computer-implemented method of the first embodiment, autoblocking the plurality of acquired ECG signals at the ECG signal provider device includes: low pass filtering the acquired ECG signals; and high pass filtering the low pass filtered ECG signals using a variable coefficient high pass filter. The variable coefficient high pass filter filters at a first frequency until an autoblocking event is detected. Upon detecting the autoblocking event, the variable coefficient high pass filter filters at a second frequency. Once the autoblocking event concludes, the variable coefficient high pass filter again filters at the first frequency.

In a tenth embodiment, in the computer-implemented method of the ninth embodiment, low pass filtering the acquired ECG signals includes a 0-524 Hz passband; the first frequency of variable coefficient high pass filter is .05 Hz; and the second frequency of variable coefficient high pass filter is 5 Hz.

In an eleventh embodiment, in the computer-implemented method of the ninth embodiment, autoblocking the plurality of transmitted first set of autoblocked Diagnostic Quality ECG signals at the first ECG signal consumer device includes: high pass filtering the transmitted first set of autoblocked Diagnostic Quality ECG signals using a variable coefficient high pass filter, and low pass filtering the high pass filtered ECG signals. The variable coefficient high pass filter filters at a third frequency until an autoblocking event is detected. Upon detecting the autoblocking event, the variable coefficient high pass filter filters at a fourth frequency. Once the autoblocking event concludes, the variable coefficient high pass filter again filters at the third frequency.

In a twelfth embodiment, in the computer-implemented method of the eleventh embodiment, low pass filtering the acquired ECG signals includes a 40 Hz passband or a 17 Hz passband for denoising; the third frequency is 0.67 Hz; and the fourth frequency is 5 Hz.

In a thirteenth embodiment, in the computer-implemented method of the first embodiment, autoblocking the transmitted first set of Diagnostic Quality ECG signals at the first ECG signal consumer device includes high pass filtering the transmitted ECG signals using a variable coefficient high pass filter and low pass filtering the high pass filtered ECG signals. The variable coefficient high pass filter filters at a first frequency until an autoblocking event is detected. Upon detecting the autoblocking event, the variable coefficient high pass filter filters at a second frequency. Once the autoblocking event concludes, the variable coefficient high pass filter again filters at the first frequency.

In a fourteenth embodiment, in the computer-implemented method of the eleventh embodiment, low pass filtering the acquired ECG signals includes a 40 Hz passband or a 17 Hz passband; the first frequency is 0.67 Hz; and the second frequency is 5 Hz.

In a fifteenth embodiment, in computer-implemented method of the first embodiment, autoblocking the plurality of acquired ECG signals includes: monitoring a select one of the ECG signals; detecting an autoblocking event in the select one of the ECG signals; and autoblocking all the ECG signals predicated on the detected autoblocking event in the select one of the ECG signals.

In the sixteenth embodiment, in the computer-implemented method of the first embodiment, autoblocking the plurality of acquired ECG signals includes: monitoring each of the ECG signals; detecting an autoblocking event on at least one of the ECG signals; and autoblocking all the ECG signals predicated on the detected autoblocking event.

In a seventeenth embodiment, in the computer-implemented method of the first embodiment, autoblocking the plurality of acquired ECG signals includes: monitoring each of the ECG signals; detecting an autoblocking event on one of the ECG signals; and autoblocking the ECG signal on which the autoblocking event is detected, the autoblocking predicated on the detected autoblocking event.

In an eighteenth embodiment, in the computer-implemented method of the first embodiment, the first set of autoblocked Diagnostic Quality ECG signals constitute a subset of the acquired Diagnostic Quality ECG signals.

In a nineteenth embodiment, in the computer-implemented method of the first embodiment, transmitting the first set of autoblocked Diagnostic Quality ECG signals includes transmitting packets of samples of the Diagnostic Quality ECG signals according to a Transmission Control Protocol/Internet Protocol ("TCP/IP").

In a twentieth embodiment, in the computer-implemented method of the third embodiment, transmitting the first set of autoblocked Diagnostic Quality ECG signals and transmitting the autoblocking indicator includes transmitting packets of samples of the Diagnostic Quality ECG signals according to a Transmission Control Protocol/Internet Protocol ("TCP/IP").

In a twenty-first embodiment, in the computer-implemented method of the nineteenth embodiment, the autoblocking of the transmitted first set of Diagnostic Quality ECG signals is performed on a packet-by-packet basis.

In a twenty-second embodiment, in the computer-implemented method of the twentieth embodiment, the autoblocking indicator is packetized with the first set of autoblocked Diagnostic Quality ECG signals.

In a twenty-third embodiment, in the computer-implemented method of the twentieth embodiment, the autoblocking indicator is transmitted separately from the packetized autoblocked first set of Diagnostic Quality ECG signals.

In a twenty-fourth embodiment, in the computer-implemented method of the third embodiment, the autoblocking indicator is transmitted as a bit stream separate from the first set of autoblocked Diagnostic Quality ECG signals.

In a twenty-fifth embodiment, in the computer-implemented method of the first embodiment, consuming the first set of Diagnostic Quality ECG signals includes performing a 12-lead reports or a S-T segment analysis.

In a twenty-sixth embodiment, in the computer-implemented method of the second embodiment, consuming the Monitor Quality ECG signals includes displaying the Monitor Quality signals.

In a twenty-seventh embodiment, a clinical care system comprises a computing system and an electrocardiogram ("ECG") signal provider device, and an ECG signal consumer device. The ECG signal provider device is programmed to: autoblock a plurality of acquired electrocardiogram signals sampled at a Diagnostic Quality rate to generate a first set of autoblocked Diagnostic Quality ECG signals; and transmit the first set of autoblocked Diagnostic Quality ECG signals from the ECG signal provider device over the computing system. The ECG signal consumer device is programmed to: obtain the transmitted first set of autoblocked Diagnostic Quality ECG signals received over the computing system; autoblock the transmitted plurality of Diagnostic Quality ECG signals at a first ECG signal consumer device to generate a plurality of Monitor Quality ECG signals; and consume at least one of the plurality of the Diagnostic Quality ECG signals and the Monitor Quality ECG signals.

In a twenty-eighth embodiment, in the clinical care system of the twenty-seventh embodiment, the programmed method of the ECG signal consumer device further comprises: autoblocking the transmitted plurality of Diagnostic Quality ECG signals at one of a plurality of ECG signal consumer devices, the plurality of ECG signal consumer devices including the first ECG signal consumer device; and utilizing the set of Monitor Quality ECG signals.

In a twenty-ninth embodiment, in the clinical care system of the twenty-seventh embodiment, the ECG provider device is further programmed to transmit an autoblocking indicator with the plurality of autoblocked Diagnostic Quality ECG signals.

In a thirtieth embodiment, in the clinical care system of the twenty-ninth embodiment, wherein the ECG provider device is further programmed to transmit the plurality of autoblocked Diagnostic Quality ECG signals and the autoblocking indicator point by point.

In a thirty-first embodiment, in the clinical care system of the twenty-ninth embodiment, the ECG provider device is further programmed to transmit the plurality of autoblocked Diagnostic Quality ECG signals and the autoblocking indicator point by point on the packet boundary.

In a thirty-second embodiment, in the clinical care system of the twenty-ninth embodiment, the ECG consumer device is further programmed to performing a 12-lead report or a S-T segment analysis from the Diagnostic Quality ECG signals.

In a thirty-third embodiment, in the clinical care system of the twenty-ninth embodiment, the ECG signal consumer device is further programmed to display the Monitor Quality signals.

In a thirty-fourth embodiment, the clinical care system of the twenty-seventh embodiment further comprising consuming the transmitted plurality of Diagnostic Quality ECG signals at a second ECG signal consumer device without autoblocking the transmitted plurality of Diagnostic Quality ECG signals.

In a thirty-fifth embodiment, in the clinical care system of the twenty-seventh embodiment, autoblocking the plurality of acquired ECG signals at the ECG signal provider device includes low pass filtering the acquired ECG signals and high pass filtering the low pass filtered ECG signals using a variable coefficient high pass filter. The variable coefficient high pass filter filters at a first frequency until an autoblocking event is detected. Upon detecting the autoblocking event, the variable coefficient high pass filter filters at a second frequency. Once the autoblocking event concludes, the variable coefficient high pass filter again filters at the first frequency.

In a thirty-sixth embodiment, in the clinical care system of the thirty-fifth embodiment, low pass filtering the acquired ECG signals includes a 0-524 Hz passband; the first frequency is .05 Hz; and the second frequency is 5 Hz.

In a thirty-seventh embodiment, in the clinical care system of the thirty-sixth embodiment, autoblocking the plurality of transmitted ECG signals at the first ECG signal consumer device includes: high pass filtering the transmitted ECG signals using a variable coefficient high pass filter and low pass filtering the high pass filtered ECG signals. The variable coefficient high pass filter filters at a third frequency until an autoblocking event is detected. Upon detecting the autoblocking event, the variable coefficient high pass filter filters at a fourth frequency. Once the autoblocking event concludes, the variable coefficient high pass filter again filters at the third frequency.

In a thirty-eighth embodiment, in the clinical care system of the thirty-seventh embodiment, low pass filtering the acquired ECG signals includes a 40 Hz low frequency cutoff or a 17 Hz low frequency cutoff; the third frequency is 0.67 Hz; and the fourth frequency is 5 Hz.

In a thirty-ninth embodiment, in the clinical care system of the twenty-seventh embodiment, autoblocking the plurality of transmitted ECG signals at the first ECG signal consumer device includes high pass filtering the transmitted ECG signals using a variable coefficient high pass filter and low pass filtering the high pass filtered ECG signals. The variable coefficient high pass filter filters at a first frequency until an autoblocking event is detected. Upon detecting the autoblocking event, the variable coefficient high pass filter filters at a second frequency. Once the autoblocking event concludes, the variable coefficient high pass filter again filters at the first frequency.

In a forty-eighth embodiment, in the clinical care system of the thirty-eighth embodiment, low pass filtering the acquired ECG signals includes a 40 Hz passband or a 17 Hz passband; the first frequency is 0.67 Hz; and the second frequency is 5 Hz.

In a forty-first embodiment, in the clinical care system of the twenty-seventh embodiment, autoblocking the plurality of acquired ECG signals includes: monitoring a select one of the ECG signals; detecting an autoblocking event in the select one of the ECG signals; and autoblocking all the ECG signals predicated on the detected autoblocking event in the select one of the ECG signals.

In a forty-second embodiment, in the clinical care system of the twenty-seventh embodiment, autoblocking the plurality of acquired ECG signals includes: monitoring each of the ECG signals; detecting an autoblocking event on at least one of the ECG signals; and autoblocking all the ECG signals predicated on the detected autoblocking event.

In a forty-third embodiment, in the clinical care system of the twenty-seventh embodiment, autoblocking the plurality of acquired ECG signals includes: monitoring each of the ECG signals; detecting an autoblocking event on one of the ECG signals; and autoblocking the ECG signal on which the autoblocking event is detected, the autoblocking predicated on the detected autoblocking event.

In a forty-fourth embodiment, in the clinical care system of the twenty-seventh embodiment, the autoblocked Diagnostic Quality ECG signals constitute a subset of the acquired Diagnostic Quality ECG signals.

In a forty-fifth embodiment, in the clinical care system of the twenty-seventh embodiment, transmitting the plurality of autoblocked Diagnostic Quality ECG signals includes transmitting packets of samples of the Diagnostic Quality ECG signals according to a Transmission Control Protocol/Internet Protocol ("TCP/IP").

In a forty-sixth embodiment, in the clinical care system of the twenty-eighth embodiment, transmitting the plurality of autoblocked Diagnostic Quality ECG signals and transmitting the autoblocking indicator includes transmitting packets of samples of the Diagnostic Quality ECG signals according to a Transmission Control Protocol/Internet Protocol ("TCP/IP").

In a forty-seventh embodiment, in the clinical care system of the forty-fifth embodiment, the autoblocking is performed on a packet-by-packet basis.

In a forty-eighth embodiment, in the clinical care system of the forty-sixth embodiment, the autoblocking indicator is packetized with the autoblocked Diagnostic Quality ECG signals.

In a forty-ninth embodiment, in the clinical care system of the forty-seventh embodiment, the autoblocking indicator is transmitted separately from the packetized autoblocked Diagnostic Quality ECG signals.

In a fiftieth embodiment, in the clinical care system of the forty-ninth embodiment, the autoblocking indicator is transmitted as a bit stream separate from the autoblocked Diagnostic Quality ECG signals.

In a fifty-first embodiment, in the clinical care system of the twenty-seventh embodiment, consuming at least one of the plurality of the Diagnostic Quality ECG signals and the Monitor Quality ECG signals comprises consuming the Diagnostic Quality ECG signals; and performing a 12-lead reports or a S-T segment analysis.

In a fifty-second embodiment, in the clinical care system of the twenty-seventh embodiment, consuming at least one of the plurality of the Diagnostic Quality ECG signals and the Monitor Quality ECG signals: comprises consuming the Monitor Quality ECG signals; rendering for display at Monitor Quality; and displaying the Monitor Quality signals.

In a fifty-third embodiment, a method for use in monitoring the physical condition of a patient is substantially as shown and described.

In a fifty-fourth embodiment, a physiological monitoring device is substantially as shown and described.

In a fifty-fifth embodiment, an electrocardiogram ("ECG") consumer device is substantially as shown and described.

In a fifty-sixth embodiment, a clinical care system is substantially as shown and described.

The expressions such as "include" and "may include" which may be used in the present disclosure denote the presence of the disclosed functions, operations, and constituent elements, and do not limit the presence of one or more additional functions, operations, and constituent elements. In the present disclosure, terms such as "include" and/or "have", may be construed to denote a certain characteristic, number, operation, constituent element, component or a combination thereof, but should not be construed to exclude the existence of or a possibility of the addition of one or more other characteristics, numbers, operations, constituent elements, components or combinations thereof.

As used herein, the article "a" is intended to have its ordinary meaning in the patent arts, namely "one or more." Herein, the term "about" when applied to a value generally means within the tolerance range of the equipment used to produce the value, or in some examples, means plus or minus 10%, or plus or minus 5%, or plus or minus 1%, unless otherwise expressly specified. Further, herein the term "substantially" as used herein means a majority, or almost all, or all, or an amount with a range of about 51% to about 100%, for example. Moreover, examples herein are intended to be illustrative only and are presented for discussion purposes and not by way of limitation.

As used herein, to "provide" an item means to have possession of and/or control over the item. This may include, for example, forming (or assembling) some or all of the item from its constituent materials and/or, obtaining possession of and/or control over an already-formed item.

Unless otherwise defined, all terms including technical and/or scientific terms used herein have the same meaning as commonly understood by one of ordinary skill in the art to which the present disclosure pertains. In addition, unless otherwise defined, all terms defined in generally used dictionaries may not be overly interpreted. In the following, details are set forth to provide a more thorough explanation of the embodiments. However, it will be apparent to those skilled in the art that embodiments may be practiced without these specific details. In other instances, well-known structures and devices are shown in block diagram form or in a schematic view rather than in detail in order to avoid obscuring the embodiments. In addition, features of the different embodiments described hereinafter may be combined with each other, unless specifically noted otherwise. For example, variations or modifications described with respect to one of the embodiments may also be applicable to other embodiments unless noted to the contrary.

Further, equivalent or like elements or elements with equivalent or like functionality are denoted in the following description with equivalent or like reference numerals. As the same or functionally equivalent elements are given the same reference numbers in the figures, a repeated description for elements provided with the same reference numbers may be omitted. Hence, descriptions provided for elements having the same or like reference numbers are mutually exchangeable.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (e.g., "between" versus "directly between," "adjacent" versus "directly adjacent," etc.).

In the present disclosure, expressions including ordinal numbers, such as "first", "second", and/or the like, may modify various elements. However, such elements are not limited by the above expressions. For example, the above expressions do not limit the sequence and/or importance of the elements. The above expressions are used merely for the purpose of distinguishing an element from the other elements. For example, a first box and a second box indicate different boxes, although both are boxes. For further example, a first element could be termed a second element, and similarly, a second element could also be termed a first element without departing from the scope of the present disclosure.

A sensor refers to a component which converts a physical quantity to be measured to an electric signal, for example, a current signal or a voltage signal. The physical quantity may for example comprise electromagnetic radiation (e.g., photons of infrared or visible light), a magnetic field, an electric field, a pressure, a force, a temperature, a current, or a voltage, but is not limited thereto.

ECG signal processing, as used herein, refers to, without limitation, manipulating an analog signal in such a way that the signal meets the requirements of a next stage for further processing. ECG signal processing may include converting between analog and digital realms (e.g., via an analog-to-digital or digital-to-analog converter), amplification, filtering, converting, biasing, range matching, isolation and any other processes required to make a sensor output suitable for processing.

Use of the phrases "capable of," "capable to," "operable to," or "configured to" in one or more embodiments, refers to some apparatus, logic, hardware, and/or element designed in such a way to enable the use of the apparatus, logic, hardware, and/or element in a specified manner. Use of the phrase "exceed" in one or more embodiments, indicates that a measured value could be higher than a pre-determined threshold (e.g., an upper threshold), or lower than a pre-determined threshold (e.g., a lower threshold). When a pre-determined threshold range (defined by an upper threshold and a lower threshold) is used, the use of the phrase "exceed" in one or more embodiments could also indicate a measured value is outside the pre-determined threshold range (e.g., higher than the upper threshold or lower than the lower threshold). The subject matter of the present disclosure is provided as examples of apparatus, systems, methods, circuits, and programs for performing the features described in the present disclosure. However, further features or variations are contemplated in addition to the features described above. It is contemplated that the implementation of the components and functions of the present disclosure can be done with any newly arising technology that may replace any of the above-implemented technologies.

The detailed description is made with reference to the accompanying drawings and is provided to assist in a comprehensive understanding of various example embodiments of the present disclosure. Changes may be made in the function and arrangement of elements discussed without departing from the scope of the disclosure. Various embodiments may omit, substitute, or add various procedures or components as appropriate. For instance, features described with respect to certain embodiments may be combined in other embodiments. In addition, descriptions of well-known functions and constructions may be omitted for clarity and conciseness. Accordingly, those of ordinary skill in the art will recognize that various changes and modifications of the examples described herein can be made without departing from the scope of the present disclosure.

Various modifications to the disclosure will therefore be readily apparent to those skilled in the art, and the generic principles defined herein may be applied to other variations without departing from the scope of the present disclosure. Throughout the present disclosure the terms "example," "examples," or "exemplary" indicate examples or instances and do not imply or require any preference for the noted examples. Thus, the present disclosure is not to be limited to the examples and designs described herein but is to be accorded the widest scope consistent with the principles and novel features disclosed.

The following numbered clauses provide additional information on the invention.
1. A computer-implemented method comprising: autoblocking a plurality of acquired electrocardiogram ("ECG") signals sampled at a Diagnostic Quality rate by an ECG signal provider device to generate a first set of autoblocked Diagnostic Quality ECG signals; transmitting the set of autoblocked Diagnostic Quality ECG signals from the ECG signal provider device over a computing system; autoblocking the transmitted set of autoblocked Diagnostic Quality ECG signals at a first ECG signal consumer device to generate a set of Monitor Quality ECG signals; and consuming at least one of the Diagnostic Quality ECG signals and the Monitor Quality signals.
2. The computer-implemented method of clause 1, further comprising: autoblocking the transmitted plurality of Diagnostic Quality ECG signals at one of a plurality of ECG signal consumer devices, the plurality of ECG signal consumer devices including the first ECG signal consumer device, to generate a set of Monitor Quality ECG signals; and consuming the set of Monitor Quality ECG signals.
3. The computer-implemented method of clause 1 or 2, wherein transmitting the first set of autoblocked Diagnostic Quality ECG signals includes transmitting an autoblocking indicator.
4. The computer-implemented method of clause 3, wherein transmitting the first set of autoblocked Diagnostic Quality ECG signals and the autoblocking indicator includes transmitting the first set of autoblocked Diagnostic Quality ECG signals and the autoblocking indicator point by point.
5. The computer-implemented method of clause 3 or 4, wherein transmitting the first set of autoblocked Diagnostic Quality ECG signals and the autoblocking indicator includes packetizing and transmitting the first set of autoblocked Diagnostic Quality ECG signals and the autoblocking indicator point by point on the packet boundary.
6. The computer-implemented method of any of clauses 3-5, wherein consuming the second set of autoblocked Diagnostic Quality ECG signals includes performing a 12-lead reports or a S-T segment analysis.
7. The computer-implemented method of any of clauses 3-6, wherein consuming the second set of autoblocked Diagnostic Quality ECG signals includes displaying the Monitor Quality signals.
8. The computer-implemented method of any of the preceding clauses, further comprising: autoblocking the transmitted first set of autoblocked Diagnostic Quality ECG signals at a second ECG signal consumer device to generate a third set of Diagnostic Quality ECG signals; and consuming the third set of autoblocked Diagnostic Quality ECG signals.
9. The computer-implemented method of any of the preceding clauses, wherein autoblocking the plurality of acquired ECG signals at the ECG signal provider device includes: low pass filtering the acquired ECG signals; and high pass filtering the low pass filtered ECG signals using a variable coefficient high pass filter, wherein: the variable coefficient high pass filter filters at a first frequency until an autoblocking event is detected; upon detecting the autoblocking event, the variable coefficient high pass filter filters at a second frequency; and once the autoblocking event concludes, the variable coefficient high pass filter again filters at the first frequency.
10. The computer-implemented method of clause 9, wherein: low pass filtering the acquired ECG signals includes a 0-524 Hz passband; the first frequency of variable coefficient high pass filter is .05 Hz; and the second frequency of variable coefficient high pass filter is 5 Hz.
11. The computer-implemented method of clause 9 or 10, wherein autoblocking the plurality of transmitted first set of autoblocked Diagnostic Quality ECG signals at the first ECG signal consumer device includes: high pass filtering the transmitted first set of autoblocked Diagnostic Quality ECG signals using a variable coefficient high pass filter, wherein: the variable coefficient high pass filter filters at a third frequency until an autoblocking event is detected; upon detecting the autoblocking event, the variable coefficient high pass filter filters at a fourth frequency; and once the autoblocking event concludes, the variable coefficient high pass filter again filters at the third frequency; and low pass filtering the high pass filtered ECG signals.
12. The computer-implemented method of clause 11, wherein low pass filtering the acquired ECG signals includes a 40 Hz passband or a 17 Hz passband for denoising; the third frequency is 0.67 Hz; and the fourth frequency is 5 Hz.
13. The computer-implemented method of any of the preceding clauses, wherein autoblocking the transmitted first set of Diagnostic Quality ECG signals at the first ECG signal consumer device includes: high pass filtering the transmitted ECG signals using a variable coefficient high pass filter, wherein: the variable coefficient high pass filter filters at a first frequency until an autoblocking event is detected; upon detecting the autoblocking event, the variable coefficient high pass filter filters at a second frequency; and once the autoblocking event concludes, the variable coefficient high pass filter again filters at the first frequency; and low pass filtering the high pass filtered ECG signals.
14. The computer-implemented method of clause 11 or 12, wherein: low pass filtering the acquired ECG signals includes a 40 Hz passband or a 17 Hz passband; the first frequency is 0.67 Hz; and the second frequency is 5 Hz.
15. The computer-implemented method of any of the preceding clauses, wherein autoblocking the plurality of acquired ECG signals includes: monitoring a select one of the ECG signals; detecting an autoblocking event in the select one of the ECG signals; and autoblocking all the ECG signals predicated on the detected autoblocking event in the select one of the ECG signals.
16. The computer-implemented method of any of the preceding clauses, wherein autoblocking the plurality of acquired ECG signals includes: monitoring each of the ECG signals; detecting an autoblocking event on at least one of the ECG signals; and autoblocking all the ECG signals predicated on the detected autoblocking event.
17. The computer-implemented method of any of the preceding clauses, wherein autoblocking the plurality of acquired ECG signals includes: monitoring each of the ECG signals; detecting an autoblocking event on one of the ECG signals; and autoblocking the ECG signal on which the autoblocking event is detected, the autoblocking predicated on the detected autoblocking event.
18. The computer-implemented method of any of the preceding clauses, wherein the first set of autoblocked Diagnostic Quality ECG signals constitute a subset of the acquired Diagnostic Quality ECG signals.
19. The computer-implemented method of any of the preceding clauses, wherein transmitting the first set of autoblocked Diagnostic Quality ECG signals includes transmitting packets of samples of the Diagnostic Quality ECG signals according to a Transmission Control Protocol/Internet Protocol ("TCP/IP").
20. The computer-implemented method of clause 3, wherein transmitting the first set of autoblocked Diagnostic Quality ECG signals and transmitting the autoblocking indicator includes transmitting packets of samples of the Diagnostic Quality ECG signals according to a Transmission Control Protocol/Internet Protocol ("TCP/IP").
21. The computer-implemented method of clause 19, wherein the autoblocking of the transmitted first set of Diagnostic Quality ECG signals is performed on a packet-by-packet basis.
22. The computer-implemented method of clause 20, wherein the autoblocking indicator is packetized with the first set of autoblocked Diagnostic Quality ECG signals.
23. The computer-implemented method of clause 20, wherein the autoblocking indicator is transmitted separately from the packetized autoblocked first set of Diagnostic Quality ECG signals.
24. The computer-implemented method of clause 3, wherein the autoblocking indicator is transmitted as a bit stream separate from the first set of autoblocked Diagnostic Quality ECG signals.
25. The computer-implemented method of any of the preceding clauses, wherein consuming the first set of Diagnostic Quality ECG signals includes performing a 12-lead reports or a S-T segment analysis.
26. The computer-implemented method of clause 2, wherein consuming the Monitor Quality ECG signals includes displaying the Monitor Quality signals.
27.A clinical care system comprising: a computing system; an electrocardiogram ("ECG") signal provider device programmed to: autoblock a plurality of acquired electrocardiogram signals sampled at a Diagnostic Quality rate to generate a first set of autoblocked Diagnostic Quality ECG signals; and transmit the first set of autoblocked Diagnostic Quality ECG signals from the ECG signal provider device over the computing system; and an ECG signal consumer device programmed to: obtain the transmitted first set of autoblocked Diagnostic Quality ECG signals received over the computing system; autoblock the transmitted plurality of Diagnostic Quality ECG signals at a first ECG signal consumer device to generate a plurality of Monitor Quality ECG signals; and consume at least one of the plurality of the Diagnostic Quality ECG signals and the Monitor Quality ECG signals.
28. The clinical care system of clause 27, the programmed method of the ECG signal consumer device further comprises: autoblocking the transmitted plurality of Diagnostic Quality ECG signals at one of a plurality of ECG signal consumer devices, the plurality of ECG signal consumer devices including the first ECG signal consumer device; and utilizing the set of Monitor Quality ECG signals.
29. The clinical care system of clause 27 or 28, wherein the ECG provider device is further programmed to transmit an autoblocking indicator with the plurality of autoblocked Diagnostic Quality ECG signals.
30. The clinical care system of clause 29, wherein the ECG provider device is further programmed to transmit the plurality of autoblocked Diagnostic Quality ECG signals and the autoblocking indicator point by point.
31. The clinical care system of clause 29, wherein the ECG provider device is further programmed to transmit the plurality of autoblocked Diagnostic Quality ECG signals and the autoblocking indicator point by point on the packet boundary.
32. The clinical care system of clause 29, wherein the ECG consumer device is further programmed to performing a 12-lead report or a S-T segment analysis from the Diagnostic Quality ECG signals.
33. The clinical care system of clause 29, wherein the ECG signal consumer device is further programmed to display the Monitor Quality signals.
34. The clinical care system of any of clauses 27-33, further comprising consuming the transmitted plurality of Diagnostic Quality ECG signals at a second ECG signal consumer device without autoblocking the transmitted plurality of Diagnostic Quality ECG signals.
35. The clinical care system of any of clauses 27-34, wherein autoblocking the plurality of acquired ECG signals at the ECG signal provider device includes: low pass filtering the acquired ECG signals; and high pass filtering the low pass filtered ECG signals using a variable coefficient high pass filter, wherein: the variable coefficient high pass filter filters at a first frequency until an autoblocking event is detected; upon detecting the autoblocking event, the variable coefficient high pass filter filters at a second frequency; and once the autoblocking event concludes, the variable coefficient high pass filter again filters at the first frequency.
36.The clinical care system of clause 35, wherein: low pass filtering the acquired ECG signals includes a 0-524 Hz passband; the first frequency is .05 Hz; and the second frequency is 5 Hz.
37. The clinical care system of clause 36, wherein autoblocking the plurality of transmitted ECG signals at the first ECG signal consumer device includes: high pass filtering the transmitted ECG signals using a variable coefficient high pass filter, wherein: the variable coefficient high pass filter filters at a third frequency until an autoblocking event is detected; upon detecting the autoblocking event, the variable coefficient high pass filter filters at a fourth frequency; and once the autoblocking event concludes, the variable coefficient high pass filter again filters at the third frequency; and low pass filtering the high pass filtered ECG signals.
38. The clinical care system of clause 37, wherein: low pass filtering the acquired ECG signals includes a 40 Hz low frequency cutoff or a 17 Hz low frequency cutoff; the third frequency is 0.67 Hz; and the fourth frequency is 5 Hz.
39. The clinical care system of any of clauses 27-38, wherein autoblocking the plurality of transmitted ECG signals at the first ECG signal consumer device includes: high pass filtering the transmitted ECG signals using a variable coefficient high pass filter, wherein: the variable coefficient high pass filter filters at a first frequency until an autoblocking event is detected; upon detecting the autoblocking event, the variable coefficient high pass filter filters at a second frequency; and once the autoblocking event concludes, the variable coefficient high pass filter again filters at the first frequency; and low pass filtering the high pass filtered ECG signals.
40. The clinical care system of clause 38, wherein: low pass filtering the acquired ECG signals includes a 40 Hz passband or a 17 Hz passband; the first frequency is 0.67 Hz; and the second frequency is 5 Hz.
41. The clinical care system of any of clauses 27-40, wherein autoblocking the plurality of acquired ECG signals includes: monitoring a select one of the ECG signals; detecting an autoblocking event in the select one of the ECG signals; and autoblocking all the ECG signals predicated on the detected autoblocking event in the select one of the ECG signals.
42.The clinical care system of any of clauses 27-41, wherein autoblocking the plurality of acquired ECG signals includes: monitoring each of the ECG signals; detecting an autoblocking event on at least one of the ECG signals; and autoblocking all the ECG signals predicated on the detected autoblocking event.
43. The clinical care system of any of clauses 27-42, wherein autoblocking the plurality of acquired ECG signals includes: monitoring each of the ECG signals; detecting an autoblocking event on one of the ECG signals; and autoblocking the ECG signal on which the autoblocking event is detected, the autoblocking predicated on the detected autoblocking event.
44. The clinical care system of any of clauses 27-43, wherein the autoblocked Diagnostic Quality ECG signals constitute a subset of the acquired Diagnostic Quality ECG signals.
45. The clinical care system of any of clauses 27-44, wherein transmitting the plurality of autoblocked Diagnostic Quality ECG signals includes transmitting packets of samples of the Diagnostic Quality ECG signals according to a Transmission Control Protocol/Internet Protocol ("TCP/IP").
46.The clinical care system of clause 27, wherein transmitting the plurality of autoblocked Diagnostic Quality ECG signals and transmitting the autoblocking indicator includes transmitting packets of samples of the Diagnostic Quality ECG signals according to a Transmission Control Protocol/Internet Protocol ("TCP/IP").
47. The clinical care system of clause 45, wherein the autoblocking is performed on a packet-by-packet basis.
48.The clinical care system of clause 46, wherein the autoblocking indicator is packetized with the autoblocked Diagnostic Quality ECG signals.
49. The clinical care system of clause 47, wherein the autoblocking indicator is transmitted separately from the packetized autoblocked Diagnostic Quality ECG signals.
50.The clinical care system of clause 49, wherein the autoblocking indicator is transmitted as a bit stream separate from the autoblocked Diagnostic Quality ECG signals.
51. The clinical care system of any of clauses 27-50, wherein consuming at least one of the plurality of the Diagnostic Quality ECG signals and the Monitor Quality ECG signals: comprises consuming the Diagnostic Quality ECG signals; and performing a 12-lead reports or a S-T segment analysis.
52.The clinical care system of any of clauses 27-51, wherein consuming at least one of the plurality of the Diagnostic Quality ECG signals and the Monitor Quality ECG signals: comprises consuming the Monitor Quality ECG signals; rendering for display at Monitor Quality; and displaying the Monitor Quality signals.
53.A method for use in monitoring the physical condition of a patient substantially as shown and described.
54.A physiological monitoring device substantially as shown and described.
55. An electrocardiogram ("ECG") consumer device substantially as shown and described.
56.A clinical care system substantially as shown and described.
57. A computer program comprising instructions which, when the program is executed by a computer, cause the computer to carry out the method of any one of clauses 1-26.

## Claims

1. A computer-implemented method comprising:
autoblocking a plurality of acquired electrocardiogram ("ECG") signals sampled at a Diagnostic Quality rate by an ECG signal provider device to generate a first set of autoblocked Diagnostic Quality ECG signals;
transmitting the first set of autoblocked Diagnostic Quality ECG signals from the ECG signal provider device over a computing system;
autoblocking the transmitted first set of autoblocked Diagnostic Quality ECG signals at a first ECG signal consumer device to generate a second set of Monitor Quality ECG signals; and
consuming at least one of the Diagnostic Quality ECG signals and the Monitor Quality signals.

2. The computer-implemented method of claim 1, further comprising:
autoblocking the transmitted first set of Diagnostic Quality ECG signals at one of a plurality of ECG signal consumer devices, the plurality of ECG signal consumer devices including the first ECG signal consumer device, to generate the second set of Monitor Quality ECG signals; and
consuming the second set of Monitor Quality ECG signals.

3. The computer-implemented method of claim 1 or 2, wherein transmitting the first set of autoblocked Diagnostic Quality ECG signals includes transmitting an autoblocking indicator, optionally wherein transmitting the first set of autoblocked Diagnostic Quality ECG signals and the autoblocking indicator includes:
transmitting the first set of autoblocked Diagnostic Quality ECG signals and the autoblocking indicator point by point; and/or
packetizing and transmitting the first set of autoblocked Diagnostic Quality ECG signals and the autoblocking indicator point by point on a packet boundary.

4. The computer-implemented method of any of the preceding claims, further comprising:
autoblocking the transmitted first set of autoblocked Diagnostic Quality ECG signals at a second ECG signal consumer device to generate a third set of Diagnostic Quality ECG signals; and
consuming the third set of autoblocked Diagnostic Quality ECG signals.

5. The computer-implemented method of any of the preceding claims, wherein autoblocking the plurality of acquired ECG signals at the ECG signal provider device includes:
low pass filtering the acquired ECG signals; and
high pass filtering the low pass filtered ECG signals using a variable coefficient high pass filter, wherein:
the variable coefficient high pass filter filters at a first frequency until an autoblocking event is detected;
upon detecting the autoblocking event, the variable coefficient high pass filter filters at a second frequency; and
once the autoblocking event concludes, the variable coefficient high pass filter again filters at the first frequency, optionally wherein:
low pass filtering the acquired ECG signals includes a 0-524 Hz passband;
the first frequency of variable coefficient high pass filter is .05 Hz; and
the second frequency of variable coefficient high pass filter is 5 Hz.

6. The computer-implemented method of claim 5, wherein autoblocking the transmitted first set of autoblocked Diagnostic Quality ECG signals at the first ECG signal consumer device includes:
high pass filtering the transmitted first set of autoblocked Diagnostic Quality ECG signals using a variable coefficient high pass filter, wherein:
the variable coefficient high pass filter filters at a third frequency until an autoblocking event is detected;
upon detecting the autoblocking event, the variable coefficient high pass filter filters at a fourth frequency; and
once the autoblocking event concludes, the variable coefficient high pass filter again filters at the third frequency; and
low pass filtering the high pass filtered ECG signals; optionally wherein
low pass filtering the acquired ECG signals includes a 40 Hz passband or a 17 Hz passband for denoising;
the third frequency is 0.67 Hz; and
the fourth frequency is 5 Hz; and
further optionally wherein the first frequency is equal to the third frequency and the second frequency is equal to the fourth frequency.

7. The computer-implemented method of any of the preceding claims, wherein autoblocking the plurality of acquired ECG signals includes:
monitoring each of the ECG signals;
detecting an autoblocking event on at least one of the ECG signals; and
predicated on the detected autoblocking event:
autoblocking all the ECG signals; or
autoblocking the at least one ECG signal on which the autoblocking event is detected.

8. The computer-implemented method of any of the preceding claims, wherein the first set of autoblocked Diagnostic Quality ECG signals constitute a subset of the acquired Diagnostic Quality ECG signals.

9. The computer-implemented method of any of the preceding claims, wherein transmitting the first set of autoblocked Diagnostic Quality ECG signals includes transmitting packets of samples of the Diagnostic Quality ECG signals according to a Transmission Control Protocol/Internet Protocol ("TCP/IP"), optionally wherein the autoblocking of the transmitted first set of Diagnostic Quality ECG signals is performed on a packet-by-packet basis.

10. The computer-implemented method of claim 3, wherein transmitting the first set of autoblocked Diagnostic Quality ECG signals and transmitting the autoblocking indicator includes transmitting packets of samples of the Diagnostic Quality ECG signals according to a Transmission Control Protocol/Internet Protocol ("TCP/IP"), optionally wherein:
the autoblocking indicator is packetized with the first set of autoblocked Diagnostic Quality ECG signals; and/or
the autoblocking indicator is transmitted separately from the packetized autoblocked first set of Diagnostic Quality ECG signals.

11. The computer-implemented method of claim 3, wherein the autoblocking indicator is transmitted as a bit stream separate from the first set of autoblocked Diagnostic Quality ECG signals.

12. The computer-implemented method of any of the preceding claims, wherein:
consuming the Diagnostic Quality ECG signals includes performing a 12-lead reports or a S-T segment analysis; and/or
consuming the Monitor Quality ECG signals includes displaying the Monitor Quality signals.

13. A clinical care system comprising:
a computing system;
an electrocardiogram ("ECG") signal provider device programmed to:
autoblock a plurality of acquired electrocardiogram signals sampled at a Diagnostic Quality rate to generate a first set of autoblocked Diagnostic Quality ECG signals; and
transmit the first set of autoblocked Diagnostic Quality ECG signals from the ECG signal provider device over the computing system; and
an ECG signal consumer device programmed to:
obtain the transmitted first set of autoblocked Diagnostic Quality ECG signals received over the computing system;
autoblock the transmitted first set of Diagnostic Quality ECG signals at a first ECG signal consumer device to generate a second set of Monitor Quality ECG signals; and
consume at least one of the plurality of the Diagnostic Quality ECG signals and the Monitor Quality ECG signals.

14. The clinical care system of claim 13, further comprising consuming the transmitted plurality of Diagnostic Quality ECG signals at a second ECG signal consumer device without autoblocking the transmitted plurality of Diagnostic Quality ECG signals.

15. The clinical care system of claim 13 or 14, wherein consuming at least one of the plurality of the Diagnostic Quality ECG signals and the Monitor Quality ECG signals comprises:
consuming the Monitor Quality ECG signals;
rendering for display at Monitor Quality; and/or
displaying the Monitor Quality signals.
